# EUROPEAN PATENT SPECIFICATION

(11) **EP 1 940 334 B1**
(45) Date of publication and mention of the grant of the patent: **01.06.2011**
(21) Application number: 06816317.9
(22) Date of filing: 04.10.2006
(51) Int. Cl.: A61F 13/58, A61F 13/56, A61F 13/60, A61F 13/62

(54) **FASTENING SYSTEMS UTILIZING COMBINATIONS OF MECHANICAL FASTENERS AND FOAMS**
BEFESTIGUNGSSYSTEME UNTER VERWENDUNG VON KOMBINATIONEN AUS MECHANISCHEN BEFESTIGUNGSELEMENTEN UND SCHAUMSTOFFEN
SYSTEMES DE FIXATION UTILISANT DES COMBINAISONS D'ATTACHES MECANIQUES ET DE MOUSSES

(30) Priority: 27.10.2005 US 260356; 27.04.2006 US 413701
(43) Date of publication of application: 09.07.2008
(73) Proprietor: KIMBERLY-CLARK WORLDWIDE, INC., Neenah, WI 54956 (US)
(72) Inventor: STABELFELDT, Sara, J., Appleton, Wisconsin 54913 (US); EFREMOVA, Nadezhda, V., Neenah, Wisconsin 54956 (US); HUANG, Yung, H., Appleton, Wisconsin 54911 (US); KRAFT, Nicholas, A., Appleton, Wisconsin 54915 (US); STEINDORF, Eric, C., Roswell, Georgia 30076 (US); YU, Lisha, Appleton, Wisconsin 54911 (US)
(74) Representative: Mabey, Katherine Frances
(86) International application number: PCT/US2006/038959
(87) International publication number: WO 2007/050253

(56) References cited:
- WO-A-02/060294
- WO-A-03/003962
- WO-A-2005/044560
- WO-A2-02/43638
- US-A- 5 614 281
- US-A1- 2004 258 902

## Description

### Background of the Invention

Traditional hook and loop mechanical fasteners are widely used in numerous products and articles such as diapers, shoes, disposable gowns, etc. In spite of their prevalence, they suffer from several drawbacks. The hook material typically is stiff and impermeable, and when used In articles worn on or near the human body, may irritate the skin or be uncomfortable. The hook material typically cannot be stretched or deformed significantly. Further, for some applications, the entanglement of hooks into loop material can frequently be difficult to remove, or may adhere to unintended surfaces. The highly abrasive nature of the hook material can also damage some surfaces. The act of peeling the hooks and loops apart can also result in a loud and unpleasant noise, making it difficult to release a fastener discreetly. Further still, in some applications low peel strength but high in-plane resistance to shear is desired, whereas conventional hook and loop fasteners may offer excessively high peel strength to achieve a given level of in-plane shear resistance.

Variations of hook and loop fasteners have been proposed in which a foam layer is used to engage with hooks, but replacing low-cost, flexible loop material with thicker, generally more expensive foams does not appear to have provided significant advantages, and does not address the known limitations of hook layers.

WO 03/003962A1 discloses an embedded mechanical fastener.

What is needed is an improved mechanical fastener that solves one or more of the aforementioned problems.

### Summary of the Invention

In response to the foregoing need, the present inventor undertook intensive research and development efforts that resulted in the discovery of an improved mechanical fastener. The present invention provides a mechanical fastener in accordance with claim 1.

The present invention provides a mechanical fastener having a flexible layer and a plurality of first discrete fastener islands having a mechanical fastening material and a backing material having a first surface attached to the mechanical fastening material and a second surface attached to the flexible layer. The mechanical fastener also has a plurality of second discrete fastener islands comprising a foam fastening layer that is attached to the flexible layer and includes a surface having a plurality of free-standing struts.

The present invention also provides a disposable absorbent article in accordance with claim 10. The disposable absorbent article having an outer cover, a bodyside liner, an absorbent core located between the bodyside liner and the outer cover, and at least one mechanical fastener. The mechanical fastener having a flexible layer, a plurality of first discrete fastener islands having a mechanical fastening material, and a backing material having a first surface attached to the mechanical fastening material and a second surface attached to the flexible layer and a plurality of second discrete fastener islands comprising a foam fastening layer that is attached to the flexible layer and includes a surface having a plurality of free-standing struts.

### Brief Description of the Drawings

FIG. 1 is a perspective view illustrating an example nonwoven fabric.
FIGS. 2A-2C are cross-section views illustrating example bicomponent strands that may be used in the nonwoven fabric shown in FIG. 1.
FIG. 3 is a perspective view illustrating another example nonwoven fabric.
FIG. 4 is a side view of an example processing line that may be used to form a nonwoven fabric.
FIG. 5 is an enlarged view illustrating a portion of an example web that may be formed using the example processing line shown in FIG. 4.
FIG. 6 is a perspective view illustrating an example fastening system.
FIG. 7 is an enlarged side view of the example fastening system shown in FIG. 6.
FIG. 8 illustrates an example absorbent article that includes the fastening system shown in FIG. 6.
FIG. 9 is an SEM photomicrograph at 50X magnification of a razor-cut cross-sectional surface of a foam layer engaged with a nonwoven fabric.
FIG. 10 is an SEM photomicrograph at 50X magnification of the surface of a foam layer.
FIG. 11 is an SEM photomicrograph at 50X magnification of the surface of a foam layer including a surface modifier.
FIG. 12 is an SEM photomicrograph at 75X magnification of a razor-cut cross-sectional surface of a foam layer including a surface modifier.
FIG. 13 depicts apparatus used for the Curved Shear Attachment Strength test.
FIG. 14 shows the geometry of a side view of a curved section of the apparatus of FIG. 13.
FIG. 15 shows another view of the apparatus used for the Curved Shear Attachment Strength test.
FIG. 16 depicts a configuration of test strips used in measuring peel strength.
FIG. 17 representatively illustrates a top plan view of an example of a mechanical fastener not in accordance with the invention.
FIG. 18 representatively illustrates a section view of the mechanical fastener of FIG. 17 along line A - A.
FIG. 19 representatively illustrates a top plan view of another example of a mechanical fastener not in accordance with the invention wherein the fastener island is attached to the flexible layer via ultrasonic bonds.
FIG. 20 representatively illustrates a section view of the mechanical fastener of FIG. 19 along line A - A.
FIG. 21 representatively illustrates a top plan view of an alternate configuration of a mechanical fastener in accordance with the invention having a plurality of fastener islands.
FIG. 22 representatively illustrates a section view of the mechanical fastener of FIG. 21 along line A - A.
FIG. 23 representatively illustrates an elevation view of an example of the fastener island.
FIG. 24 representatively illustrates a plan view of a disposable absorbent article including mechanical fasteners, where the absorbent article is shown in a stretched and laid flat condition with the surface of the article which contacts the wearer's skin facing the viewer and with portions of the article partially cut away to show the underlying features.
FIG. 25 representatively illustrates a top plan view of an alternate configuration of a mechanical fastener having discrete fastener islands in accordance with the invention and a flexible layer that is extensible between the fastener islands.
FIG. 26 representatively illustrates a top plan view of two alternate configurations of mechanical fasteners in accordance with the invention having different arrangements of fastener islands to provide different lines of flexure.

### Definitions:

As used herein, a foam material is **"open-celled"** if at least 60% of the cells in the foam structure that are at least 1 micrometer (µm) in size are in fluid communication with at least one adjacent cell. In one embodiment, at least 80% of the cells in the foam structure that are at least 1 µm in size are in fluid communication with at least one adjacent cell.

As used herein, the term **"strand"** refers to an elongated extrudate formed by passing a polymer through a forming orifice (e.g., a die). A strand may include a fiber, which is a discontinuous strand having a definite length, or a filament, which is a continuous strand of material.

As used herein, the term **"reticulated foam"**, as it is commonly used among those skilled in the art, denotes solid foamed materials where substantially all intervening "window walls" or cell membranes have been removed from the cells of the foam, leaving a network consisting primarily of interconnected struts along the outlines of the cells formed during the foaming.

Reticulated foams are thus distinct from foams in which the window walls are merely broken, or foams in which only the outermost window walls or skin have been removed by physical means. Reticulated foams, by virtue of their general lack of cell membranes, are highly permeable to gas and liquid alike, offering little resistance to fluid flow, indeed much less than those foams in which the cell membranes have been retained.

Reticulation is typically achieved by known foam processing procedures applied to the foam after the cells have been formed. These procedures may involve the use of caustic treatments (e.g., see U.S. Patent No. 3,266,927, issued to Fritz et al. on August 16, 1966), attack by other reactive compounds such as ozone, or thermal treatments of the foam, removing all or substantially all of the "window walls" separating the cells throughout the foam. In some cases, other treatments such as controlled explosions are used to remove membranes around portions of cells (for example, a foam may be packed into an explosion chamber containing an explosive gaseous medium which is then exploded). An example of explosive treatment of a foam is given in US Pat. No. 4,906,263, issued to von Blucher et al. on March 6, 1990.

Needling may also be used to open a closed cell foam material, as described in U.S. Patent No. 4,183,984, issued to Browers et al. on January 15, 1980. Other methods for creating an open cell foam material are disclosed in U.S. Patent No. 6,720,362, issued to Park et al. on April 13, 2004.

In one embodiment, reticulation is only present in the outer portions of a foam layer at and near the engaging surface.

Alternatively, the cellular foam material may be inherently reticular as made. According to U.S. Patent No. 3,661,674, issued to Higgs et al. on May 9, 1972, an inherently reticular polyester polyurethane foam may be made, for example, by allowing the foam-forming ingredients to react in the presence of a viscosity-retarding substance such as a further polyester having an acid component which is the same as that of the polyester used to make the foam material but which has a hydroxyl number of between 10 and 100 and a viscosity of less than 200 poises.

As used herein, the term **"stretchable"** refers to materials which, upon application of a stretching force, can be extended to a stretched dimension which is at least 150% of an original dimension (i.e., at least 50% greater than an original, unstretched dimension) in one or more directions without rupturing. The term "elastic" refers to materials which are stretchable and which, upon release of the stretching force, will retract (recover) by at least 50% of the difference between the stretched dimension and the original dimension. For instance, a material having an original dimension of 20 cm is stretchable if it can be extended to a dimension of at least 30 cm without rupture. The same material is elastic if, after being extended to 30 cm, it retracts to a dimension of 25 cm or less when the stretching force is removed.

As used herein, the term **"Denier"** refers to a weight-per-unit-length measurement of a linear material defined as the number of grams per 9000 meters. The term may refer to either an individual fiber or a bundle of fibers (yarn).

As used herein, **"Decitex"** (abbreviated "dtex") is a term similar to denier except it is the weight in grams of 10,000 meters of a yarn or fiber.

As used herein, the term **"hydroentangling"** refers to techniques of treating a fabric by application of high-velocity jets of water delivered from high-pressure orifices, whereby the fibers or filaments in the fabric are rearranged under the influence of water impingement. By way of example, U.S. Patent No. 3,485,706, issued to Evans on December 23, 1969, discloses a hydroentanglement process for manufacture of nonwoven fabric webs. During hydroentanglement, the nonwoven fabric web is typically positioned on a foraminous forming surface as it is subjected to impingement by the water jets, whereby the fibers or filaments of the nonwoven fabric web become entangled, thus creating a nonwoven fabric web with coherency and Integrity, while the specific features of the forming surface act to create the desired pattern in the nonwoven fabric web. Before leaving the nozzles, the water may have a pressure of up to about 60 Mpa (600 bar). The nozzles may have a diameter of 0.05 to 0.25 mm and may be spaced at 20-160 mesh. The jet hits the nonwoven fabric web surface, penetrates it and flows to the openings in the foraminous surface (the web support) and through suction slots. In this process, the fibers are entangled, which may cause compacting and bonding of the nonwoven fabric web. See also, U.S. Patent No. 5,389,202, issued to Everhart et al. on February 14, 1995.

The foraminous surface may be substantially planar or three-dimensional, and may be a perforated metal surface, a metal wire, a polymeric wire or fabric such as a through-drying fabric known in papermaking, or other surface. Related examples of hydroentanglement technology are found, by way of examples, in U.S. Patent No. 4,805,275, issued to Suzuki et al. on February 21, 1989, where three-dimensional foraminous surfaces are disclosed. See also U.S. Patent Application 2002/0025753, published by Putnam et al. on February 28, 2002.

As used herein, the phrase **"cluster of free-standing struts"** refers to one or more interconnected struts that extend away from a complete cell of the foam material, wherein the struts in the cluster are connected to the same complete cell. If first and second struts from first and second cells, respectively, join at a juncture and have a third strut (a free-standing strut) extending from the juncture, the first and second struts are considered to be part of a closed cell, and the cluster of free-standing struts would consist of the third strut. If the third strut branches into two other free-standing struts at an end away form the juncture, the third strut and the two other free-standing struts are all part of a cluster of free-standing struts.

As used herein, the term **"free length"** of a free-standing strut or cluster of free-standing struts is the linear distance the free-standing strut or cluster of free-standing struts, respectively, extends away from the nearest portion of the first complete cell in the foam material attached to the free-standing strut or cluster of free-standing struts.

### The Foam Layer

In one embodiment, the foam layer comprises an open-celled foam such as a melamine foam, a polyurethane foam, or other known open-celled foams. Such foam materials typically comprise rod-like struts forming a reticulated network that defines cells in the foam materials.

Melamine-based foams may include the foams currently manufactured by BASF, located in Ludwigshafen, Germany, under the BASOTECT® brand name. For example, BASOTECT® 2011, with a density of about 0.01 g/cm³, may be used. Blocks of melamine-based foam are marketed by Procter & Gamble, located in Cincinnati, Ohio, under the MR. CLEAN® brand name. Similar materials are marketed under the CLEENPRO™ name by LEC, Inc., located in Tokyo, Japan, (several product executions are shown at http://www.users.bigpond.com/jmc.au/CLEENPRO/CLEENPRO-E.htm and http://www.users.bigpond.com/jmc.au/CLEENPRO/CLEENPRO%20Family-E.htm, both printed on November 13, 2003). Melamine-based foam is also marketed for acoustic and thermal insulation by many companies such as American Micro Industries, located in Chambersburg, Pennsylvania.

Examples of potentially useful reticulated foams include the polyurethane reticulated foams of Foamex, Inc., located in Linwood, Pennsylvania, such as foam SIF-60z; and, the reticulated foams of the following firms: Crest Foam Industries, Inc., located in Moonachie, New Jersey, including FilterCrest® reticulated foams; Scottfoam Corporation, located in Eddystone, Pennsylvania; Swisstex, Inc., located in Greenville, South Caroline; Recticell, located in Chicago, Illinois; and, the foams produced at Caligen Europe BV, located in Breda, the Netherlands, a subsidiary of British Vita PLC, located in Manchester, England.

Examples of reticulated foams are also disclosed in the patent literature, including U.S. Patent No. 3,171,820, issued to Volz et al. on March 2, 1965; U.S. Patent No. 4,631,077, issued to Spicer et al. on December 23, 1986; U.S. Patent No. 4,656,196, issued to Kelly et al. on April 7, 1987; and, U.S. Patent No. 4,540, 717 issue to Mahnke et al. on September 10, 1985. Also of potential use are the open-celled foams marketed by Sydney Heath & Son, located in Burslem, Stoke on Trent, United Kingdom, including reticulated foam described as having 75 pores per inch. Reticulated foams may include polyurethane, polyester, and polyether types, as well as other known reticulated foams. Other foams that may be considered include those of U.S. Patent No. 4,062,915, issued to Stricharczuk et al. on December 13, 1977.

Pore size in commercial open-celled foams is commonly expressed as pores per inch (ppi), based on measurement of the pores along a straight path of known length, which may also be expressed in terms of pores per centimeter (ppc). According to the present invention, the foam material in the foam layer may have an characteristic pore size of any of the following: from about 1 ppc to about 200 ppc; from about 3 ppc to about 180 ppc; from about 10 pc to about 150 ppc; from about 15 ppc to about 130 ppc; from about 15 ppc to about 100 ppc; or, from about 20 ppc to about 65 ppc.

The free-standing struts of the foam material, by way of example only, may have an effective diameter of about 0.3 microns or greater, such as about 1 micron or greater, about 3 microns or greater, or about 10 microns or greater, such as any of the following: from about 0.3 micros to about 30 microns; from about 1 micron to about 30 microns; from about 3 microns to about 30 microns; from about 1 micron to about 20 microns; and, from about 1 micron to about 10 microns. The free length of a free-standing strut, the free length of a plurality, or cluster, of free-standing struts effective in engaging a landing layer, the free length of a characteristic free-standing strut, the average free length of free-standing struts on a surface of a foam material, or the median free length of free-standing struts on a surface of a foam material, may be any of the following: greater than about 3 microns; greater than about 10 microns; greater than about 20 microns; greater than about 50 microns; greater than about 100 microns; greater than about 500 microns; greater than about 1000 microns; and, greater than about 2000 microns, such as from about 10 microns to about 2000 microns, or from about 50 microns to about 1000 microns, or from about 100 microns to about 500 microns. The ratio of free length of a free-standing strut (or related measures thereof previously discussed) to effective diameter of a free-standing strut may be about 5 microns or greater, 10 microns or greater, 20 microns or greater, 50 microns or greater, and 100 microns or greater, such as from about 5 microns to about 100 microns, or from about 10 microns to about 200 microns.

Other open-celled foam materials may also be considered, such as a layer of an aminoplast foam (e.g., foams made from urea-formaldehyde resins or melamine-formaldehyde resins), a phenolic foam such as a foam made from phenolformaldehyde resins. Any aminoplast foam or other open-celled foam disclosed in U.S. Patent No. 4,125,664, issued to Giesemann on November 14, 1978, the disclosure of which is incorporated by reference to the extent that it is non-contradictory herewith, may be used to produce the articles of the present invention. Other foams that may be used within the scope of the present invention include those disclosed in U.S. Patent No. 4,666,948, issued to Woemer et al. on May. 19, 1987; U.S. Patent No. 5,234,969, issued to Clark et al. on August 10, 1993; U.S. Patent No. 6,133,332, issued to Shibanuma on October 17, 2000; and, World Patent Application No. WO 91/14731, published by Mäder et al. on October 3, 1991.

In one embodiment, the foam layer comprises a thermoset foam, and the thermoset components of the foam layer may comprise over 50%, over 60%, over 80%, or over 90% of the mass of the foam layer. Alternatively, the solid polymeric components of the foam layer may consist essentially of one or more thermoset materials. In another embodiment of the present invention, the foam layer may be substantially free of thermoplastic materials. In another embodiment of the present invention, the foam layer may not comprise more than 50% of any one of a component selected from polyolefin materials, polyurethanes, silicones, and polyesters.

The foam layer may comprise more than one kind of foam. For example, heterogeneous foam layers may be considered with structures or compositions similar to any of those disclosed in U.S. Patent No. 5,817,704, issued to Shiveley et al. on October 6, 1998. Two or more kinds of foam material may be blended or joined together during foam manufacture or existing foams may be laminated or otherwise joined together.

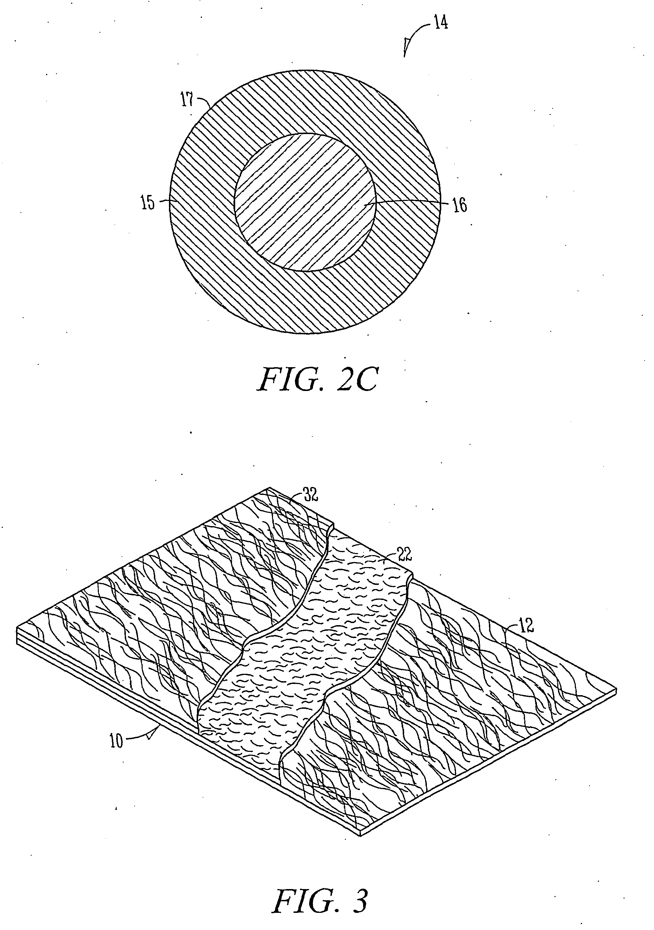

The foam layer may be cut or sliced to any desired thickness, and may be cut to be planar, sinusoidal, or to have other geometric features. Principles for cutting and slicing a foam layer are disclosed in European Patent No. EP 191,475. published by Gotoh et al. on August 20, 1986; U.S. Patent No. 5,670,101, issued to Nathoo et al. on September 23, 1997, which shows a slicer (object no. 32 in Figure 3) that slices foam material into multiple layers at once, presumably by the action of multiple cutting blades; and, U.S. Patent No. 6,245,697, issued to Conrad et al. on June 12, 2001, which discloses the use of a sharp reciprocating saw blade to slice a foam material into thin layers, such as from about 0.5 mm to about 5 mm in thickness.

Another method for slicing foam material to thin small layers (e.g., about 1 mm in thickness or greater) is found in Japanese Patent Application No. JP 2001-179684A, published by Toshiro on July 3, 2001, which discloses joining a reinforcing layer to a foam material prior to slicing to allow the thin layer to be processed more easily. The foam material with a reinforcing layer is compressed in a nip and then encounters a blade that severs a thin layer away from the main body of the foam material. By extension to the present invention, a reinforcing layer, such as a nonwoven web or paper towel, may be adhesively joined to a thick block of foam material, and then pass through a nip and encounter a knife blade oriented to slice away a thin section of foam material attached to the reinforcing layer. The remaining thicker block of foam material could then again be attached to a second reinforcing layer on one side, and the foam material adjacent to the reinforcing layer could be sliced off, as before, and the process could be repeated until the foam material had been substantially cut into a plurality of thin layers attached to a reinforcing layer. Both sides of the initial foam material block may be attached to a reinforcing layer, if desired, optionally allowing the final split to divide a foam material into two thin layers both attached to reinforcing layers.

In addition to being sliced from larger foam material blocks, the foam material may be formed directly in thin layers using methods such as those disclosed in World Patent Application No. WO 98/28118, published by Peterson et al. on July 2, 1998.

The foam material may also be perforated, as may the reinforcing layer. One method for perforating foam materials is disclosed in World Patent Application No. WO 00/15697, published by Park et al. on March 23, 2000. The foam material may also have a plurality of short slits or elongated perforations applied normal to the plane of the foam material, such as the slit materials in U.S. Patent No. 5,397,316, issued to LaVon et al. on March 14, 1995.

### Reinforcing Layer:

The foam layer may be reinforced with an underlying reinforcing layer such as a nonwoven web, a tissue web, a woven fabric, a scrim material, and the like. In one embodiment of the present invention, the reinforcing layer may generally comprise cellulosic fibers and may comprise a paper material such as a latex-reinforced creped towel, an uncreped through-air-dried towel reinforced with wet strength resins or other binding agents, other single-ply or multi-ply tissue structures (multi-ply tissues may generally require interply bonding means such as adhesive attachment for good mechanical integrity), a coform layer comprising wood pulp fibers intermingled with thermoplastic material that has been thermally bonded (e.g., by application of heated air, heated calendering, etc.), and airlaid material comprising bicomponent binder fibers, a hydroknit comprising hydraulically entangled paper fibers on a nonwoven substrate, and the like. The reinforcing layer, such as a web, may comprise a plurality of layers bonded together.

Foam layers joined to reinforcing layers are disclosed in commonly owned U.S. Patent (Publication No. 2005-0138238A1) Application Serial Number 10/744,238, filed by Chen et al. on December 22, 2003. While the products of the Chen et al. application are primarily intended to serve as cleaning devices, the combinations of foam layers and reinforcing layers disclosed therein may be adapted for the present invention.

The reinforcing layer may be coextensive with the foam layer, or may extend across only a portion of the foam layer, or may extend beyond all or any of the lateral sides of the foam layer.

Attachment of the reinforcing web to the foam material may be accomplished by adhesive means suitable for maintaining good flexibility in the article. In addition, the adhesive means may also provide good strength under humid or wet conditions and the stresses typical during use of the article. In one embodiment of the present invention, the adhesive means comprises a water-insoluble hot melt adhesive material having a Shore A hardness of about 95 or less, specifically about 75 or less, more specifically about 55 or less, more specifically still about 40 or less, and most specifically about 30 or less, such as from about 10 to about 95, or from about 20 to about 55. Useful adhesive materials may include, but are not limited to those disclosed in U.S. Patent No. 6,541,679, issued to Betrabet et al. on April 1, 2003 and U.S. Patent No. 5,827,393, issued to Kinzelmann et al. on October 27, 1998, as well as the commercial HYSOL® hotmelts of Henkel Loctite Corporation, located in Rocky Hill, Connecticut, as well as polyolefin, urethane, and polyamide hotmelts. The adhesive material may have a glass transition temperature between about -10°C and about +30° C. or between about 10°C. and about 25°C. The tensile strength of the adhesive material may be at least about 100kPa (100 psi), at least about 2068 kPa (300 psi), or at least about 3447 kPa (500 psi).

In one embodiment of the present invention, the adhesive means may comprise an adhesive material with a plurality of hydrophilic groups suitable for maintaining good adhesion with cellulose material even when the cellulose material is wet. Such adhesive materials may comprise EVA (ethylene vinyl acetate), and may include, by way of example, the EVA HYSOL® hotmelts commercially available from Henkel Loctite Corporation, located in Rocky Hill, Connecticut, including 232 EVA HYSOL®, 236 EVA HYSOL®, 1942 EVA HYSOL®, 0420 EVA HYSOL® SPRAYPAC®, 0437 EVA HYSOL® SPRAYPAC®, CoolMelt EVA HYSOL®, QuikPac EVA HYSOL®, SuperPac EVA HYSOL®, and WaxPac EVA HYSOL®. EVA-based adhesive materials may be modified through the addition of tackifiers and other conditioners, such as Wingtack 86 tackifying resin manufactured by Goodyear Corporation, located in Akron, Ohio.

In another embodiment of the present invention, the adhesive means comprises an elastomeric adhesive material such as a rubber-based or silicone-based adhesive material, including silicone sealants and latex adhesive materials such as acrylic latex. In one embodiment of the present invention, however, the adhesive means is substantially free of natural latex or proteins associated with natural latex. In another embodiment of the present invention, the adhesive means is substantially free of any kind of latex.

The adhesive means may also comprise fibers or particulates that are either tacky or may be heated to melt a portion thereof for fusing a fibrous web to the foam layers. For example, bicomponent binder fibers may be used, in which the fibers include a sheath having a lower melting point than a core fiber (e.g., a polypropylene or polyethylene sheath around a polyester core). The binder fibers may be applied in a separated loose form, or may be provided as a prebonded fusible web. In one embodiment of the present invention, the adhesive means comprises a combination of adhesive particles or fibers such as bicomponent binder fibers and a hotmelt or reactive adhesive material. For example, bicomponent binder fibers may be present in or on a reinforcing layer prior to application of a hotmelt or other flowable or liquid adhesive (e.g., by spray, extrusions, or printing) to either the reinforcing layer or the foam, followed by joining of the reinforcing layer to the foam layer and optional application of heat or other curing means. The particulate adhesive component may already be active (e.g., partially molten) when the foam is joined to the reinforcing layer.

In general, the adhesive means may be applied by spray nozzles, glue guns, bead applicators, extruders, gravure printing, flexographic printing, ink-jet printing, coating, and the like. The adhesive means may be, but need not be, uniformly applied on either the surface of the foam layer or the surface of the reinforcing layer or both, and may be applied selectively in regions where high strength is needed such as along the perimeter of the interfacial area between the reinforcing layer and the foam layer. The adhesive means may also be applied in a pattern or in a substantially random distribution.

The foam layer may have a thickness about 1 mm to about 15 mm, from about 2 mm to about 12 mm, from about 3 mm to about 10 mm, and from about 4 mm to about 8 mm. The ratio of the thickness of the reinforcing layer to the thickness of the foam layer may be any of the following: from about 1 to about 200; from about 3 to about 10; from about 4 to about 10; from about 0. 2 to about 2; from about 0.3 to about 2; from about 0. 3 to about 1; less than about 1; greater than about 1; and, from about 0. 5 to about 1.5.

The reinforcing layer joined to the foam layer may be a nonwoven web, a tissue web, a film, an apertured web, a laminate, and the like. Suitable nonwoven webs may include meltblown webs, spunbond webs, spunlace webs, and the like. The reinforcing layer may be elastomeric, such as the webs disclosed in U.S. Patent No. 4,707,398, issued to Boggs on November 17, 1987; U.S. Patent No. 4,741,949, issued to Morman et al. on May 3, 1988; and, U.S. Patent No. 5,520,980, issued to Morgan et al. on May 28, 1996. The reinforcing layer may be a neck-bonded laminate or other stretchable laminate.

Alternatively, a foam layer may be produced such that a reinforcing layer is unitary with the foam material itself. For example, a single layer of foam material may be produced with a skin on one side that may reinforce the foam material. Similarly, a foam layer may have substantially closed cells on one side and substantially open cells on the other side. Such a foam layer may be an example of a "gradient foam material" having a gradient in the thickness direction pertaining to a material property such as pore size, openness of the pores, density, etc. Gradient foam materials comprising one side providing a reinforcing function may be produced from foams having a skin on one side or from closed-cell foam materials in which one surface is converted to an open-cell foam material through chemical or mechanical means to remove windows from the foam material and liberate free-standing struts on one surface.

Further, the foam layer may also comprise adhesive material to further enhance bonding of the foam material to a landing layer. The adhesive material may be provided on a tab or extension of a reinforcing layer such that the adhesive treated zone is not on the foam material itself but on an attached portion of another material, or the adhesive material may be present on the surface or within the body of the foam material. In one embodiment of the present invention, viscous adhesive material is present within the foam material but not necessarily on the surface of the foam material, such that adhesive attachment does not occur when the foam material contacts another material unless the foam material is loaded sufficiently to bring the internal adhesive into contact with the other material (e.g., a landing layer). Pressure sensitive adhesive material may be sprayed on the surface of a foam material, or injected or impregnated into the foam material to form spaced-apart deposits within the foam material. An adhesive section attached to a foam layer may be shielded with release paper or other means to prevent premature attachment.

In another embodiment of the present invention, the addition of adhesive means to a foam layer fastening system may help increase the peel strength of the foam layer fastening system, when higher peel is desired.

### The Landing Material

The landing material for use in the landing layer of the present invention may be a loop material known in past hook and loop systems, though for best results the size of the loops or holes in the landing layer should be adjusted for effective attachment with the foam layer to be used. The loop material may be a web comprising hook-engageable, free-standing loops extending from at least one surface of the loop material.

The landing material may be a nonwoven web such as a meltspun (meltblown or spunbond web), a needled fibrous web, or a hydroentangled web (e.g., a spunlace web, particularly one with microfibers hydroentangled onto a base fabric). The landing layer may comprise fibrous loops that rise away from the plane of the fabric or lie in the plane of the fabric, making it possible for the loops to be engaged by a suitable opposing surface having free-standing struts of the foam layer.

It has been found that good results may be obtained when the landing layer has numerous loop segments rising from the surface of the fabric with a characteristic loop height greater than about 30 microns, such as about 50 microns or greater, about 80 microns or greater, about 100 microns or greater, or about 150 microns or greater, which may span characteristic ranges such as from about 30 microns to 1000 microns, or from about 50 microns to 700 microns, or from about 80 microns to about 600 microns, or from about 100 microns to about 500 microns. The linear distance on the surface of the fabric between the two ends of an elevated loop segment (or the distance between the points where the loop segments return to the plane of the fabric) may be about 80 microns or greater, such as about 150 microns or greater, about 300 microns or greater, or about 500 microns or greater, with characteristic ranges such as from about 80 microns to about 1000 microns, or from about 100 microns to about 800 microns, or from about 100 microns to about 600 microns. However, other size ranges are also within the scope of the present invention and may be considered, provided that the free-standing struts of the engaging surface of a foam layer are capable of adequate engagement with the loop segments or holes on the engaging surface of the landing layer.

In one embodiment of the present invention, the landing layer comprises loop segments comprising microfibers having an effective fiber diameter of about 30 microns or less, about 20 microns or less, about 10 microns or less, about 5 microns or less, about 2 microns or less, or about 1 micron or less. The fiber diameters of the microfibers may range from about 0.1 micron to about 30 microns, or from about 1 micron to about 30 microns, or from about 1 micron to about 20 microns, or from about 2 microns to about 20 microns. Such microfibers may be produced by known meltblown processes, for example. Bicomponent meltblown fibers, as used herein includes other multi-component conjugate fibers, may be used to obtain extremely fine fibers by splitting the fibers or removing one of the components. Splitting may be done by mechanical or chemical means. For example, a bicomponent side-by-side or pie-segment type fiber may be split using hydroentanglement using high-velocity jets of water to split the multi-component fibers. Chemical treatment to cause swelling of a component (e.g., by application with caustic or other swelling agents) or to dissolve a component may also result in splitting. Steam treatment, microwaves, mechanical straining, and other techniques may also be applied to suitable mutli-component fibers to promote splitting. The bicomponent fibers may be round in cross-section or non-round, such as multilobal fibers, and may be twisted, crimped, helical, or substantially straight. Bicomponent combinations, by way of example only, may include any of the following: polypropylene, polyethylene, polyesters, PBT (polybutyleneterephthalate), polylactic acids, polyamides, PHA, and the like. Additional details on microfiber production are found in U.S. Patent Application Publication No. 2004/0161994 A1, published by Arora et al. on August 19, 2004; the microfibers of the Arora et al. document may also be used within the scope of the present invention.

A landing layers comprising microfibers may be woven textiles or nonwoven fabrics, and may comprise a single type of microfibers or a plurality of microfibers types, and may comprise fibers, webs, or other structural elements others than microfibers. Exemplary materials comprising microfibers that may be considered for use in a landing layer according to the present invention include the following:
- Spunlace webs, particularly those comprising microfibers, as manufactured by Polymer Group, Inc. (located at North Charleston, South Carolina). Patents and applications assigned to Polymer Group, Inc. (PGI) that involve hydroentangling include U.S. Patent Application Publication No. 2002/0025753, published by Putnam et al. on February 28, 2002; U.S. Patent No. 6,306,234, issued to Barker et al. on October. 23, 2001; U.S. Patent No. 6,314,627, issued to Ngai et al. on November 13, 2001; U.S. Patent Application Publication No. 2002/0146957, published by Fuller et al. on October 10, 2002; U.S. Pat. No. 6,675,429, issued to Carter et al. on January 13, 2004; U.S. Patent No. 6,606,771, issued to Curtis et al. on August 19, 2003; U.S. Patent No. 6,564,436, issued to Black et al. on May 20, 2003; U.S. Patent No. 6,516,502, issued to Moody et al. on February 11, 2003; U.S. Pat. No. 6,725,512, issued to Carter et al. on April 27, 2004; U.S. Pat. No. 6,735,833, issued to Putnam et al. on May 18, 2004; and, U.S. Patent No. 6,343,410, issued to Greenway et al. on February 5, 2002, the disclosures of which are each incorporated by reference to the extent that they are non-contradictory herewith. Commercial PGI products that may be used in various embodiments of the present invention include PGI's MediSoft™ fabrics, Comfortlace™ fabrics for feminine hygiene products, said to be made with PGI's Laminar Air Controlled Embossing (LACE) process that adds a 3-D image or bulky surface layer to a reticulated film, and Miratec™ fabrics or other fabrics made with PGI's Apex® hydroentanglement technology in which a 3-D image may be added to a fabric.
- Looped material wherein the loops are formed in a landing layer according to U.S. Patent Application Publication No. 2004/0157036A1, published by Provost et al. on August 12, 2004. The loop material is formed by needling a batt of fibers through a carrier sheet such as a plastic film, to form loops on the opposing side of the carrier sheet. A binder, such as a powder resin or plastic film, is placed over the fiber side of the product and fused to the carrier sheet to bond the fibers in place. In some cases the product is needled in only discrete areas, leaving other areas free of loops.
- Apertured nonwoven webs made according to U.S. Patent No. 5,369,858, issued to Gilmore et al. on December 6, 1994. This patent document is a nonwoven fabric comprising at least one layer of textile fibers or net of polymeric filaments and at least one web of melt blown microfibers, bonded together by hydroentangling. The nonwoven fabric may be apertured by hydroentangling or may have areas of higher density and areas of lower density. The technology is assigned to Fiberweb North America located in Simpsonville, South Carolina.
- Microfiber cloths marketed as cleaning cloths, such as Modern Magic® MicroFiber Cleaning Cloths by Modern Plastics, Inc. located in Bridgeport, Connecticut; the MicroFiber Cleaning Cloths of TAP Plastics, Inc. located in Stockton, California; or, the Scoth-Brite® MicroFiber Cleaning Cloths of 3M, Inc. located in St. Paul, Minnesota.
- OFO-3 Micro Fiber made by Oimo Industrial Co., Ltd., located in Taipei, Taiwan, a cloth made of mechanically split microfiber made from a PET/nylon bicomponent fiber that is hydraulically needled, splitting the fiber into 166 parts, according to supplier information at http://www.allproducts.com/household/oimo/22-ofo-3.html (viewed on May 17, 2004).

Microfibers may be made from numerous polymers such as cellulose (e.g., lyocell solvent-spun fibers), polyolefins, polyamides, polyesters, PHA, polylactic acid, acrylic, and the like. Microfibers may also include electrospun fibers, which are also referred to as nanofibers.

Known loop materials that may be adapted for use in a landing layer of the present invention include the loop materials disclosed in U.S. Patent No. 5,622,578, issued to Thomas on April 22, 1997. The loops, as disclosed in the patent document, are manufactured by the process of extruding liquid material through the apertures of a depositing member onto a moving substrate to form the base of the loop, stretching the liquid material in a direction parallel to the plane of the substrate, severing the stretched material to form a distal end which fuses with an adjacent amount of stretched material to form a loop.

Loop materials that may be adapted for use in a landing layer of the present invention may include laminates of nonwoven materials, such as nonwoven webs joined to films or multiple layers of fibrous nonwoven webs. Such laminated may include those disclosed in U.S. Patent Application Publication No. 2003/0077430, published by Grimm et al. on April 24, 2003. The laminates disclosed in Grimm et al. document comprise at least one layer of a polyolefin endless filament nonwoven fabric, such as a polypropylene endless filament nonwoven fabric, having a maximum tensile strength in the machine running direction that is at least as great as crosswise to that direction (e.g., in a ratio of about 1:1 to about 2.5:1), and made up essentially of fibers having a titer of less than about 4.5 dtex, such as in the range of about 0.8 dtex to about 4. 4 dtex, more specifically from about 1.5 dtex to about 2.8 dtex, as well as a second layer of a nonwoven fabric that is bonded to the first layer, which includes a sheet of crimped, such as two-dimensionally and/or spirally crimped, staple fibers made of polyolefins, and whose crimped fibers are coarser than the fibers of the nonwoven fabric of the first layer, and can have titer of about 3.3 dtex to about 20 dtex, more specifically about 5.0 dtex to about 12.0 dtex, whereby the at least two nonwoven fabric layers may be bonded to one another at the common interface by bonding in the form of a predetermined pattern. The second layer can act as the loop layer in the material of the Grimm et al. document.

Alternatively, the landing layer of the present invention may comprise openings (holes) that may be engaged by free-standing struts in a foam layer. The openings may be pores in the surface of the landing layer defined by surrounding fibers. Such openings may have a characteristic diameter greater than about 0.5 microns (µm), such as from about 0.5 µm to about 3 millimeters (mm), or from about 1 µm to about 2 mm, or from about 2 µm to about 1.2 mm, or from about 4 µm to about 1 mm, or less than about 1 mm. The openings may maintain an effective diameter of about 0.5 microns or greater, about 1 micron or greater, about 2 microns or greater, or about 4 microns or greater, continuously from the surface plane of the landing layer surrounding the opening to a "hole depth" in the landing layer of about any of the following or greater: 2 microns, 5 microns, 10 microns, 50 microns, 100 microns, 300. microns, 600 microns, 1 mm, 2 mm, and 3 mm. If the opening provides a continuous vertical opening adapted to receive a vertically oriented cylindrical free-standing strut of diameter D extending a maximum distance L into the landing layer, the opening may have a Cylindrical Hole Depth of L with respect to a free-standing strut diameter of D. Thus, for an example, a free-standing strut having a maximum diameter of about 50 microns and a height of about 500 microns relative to its base (the region where it connects to two or more other struts) should be able to penetrate about 300 microns into a substantially flat landing layer with openings having a Cylindrical Hole Depth of about 300 microns with respect to a free-standing strut diameter of about 50 microns.

In one embodiment of the present invention, the landing layer comprises fine microfibers that may provide loop elements to engage the free-standing struts of the foam layer. In another embodiment of the present invention, the microfibers are provided in a spunlace web in which microfibers have been hydroentangled on a nonwoven or woven backing layer.

In one alternative embodiment of the present invention, the landing layer may also comprise an open-celled foam material, such as a melamine-based foam layer. It has been found that one foam layer of melamine foam material may engage effective, under some circumstances, with another foam layer of melamine foam material, for the open cells and cell windows of a melamine foam material structure may serve as loops suitable for engaging free-standing struts from another foam layer. In such an embodiment, the foam layer or the landing layer comprising a foam layer may each further comprise a reinforcing layer.

### Manufacture of Melamine Foam

Principles for manufacturing melamine-based foam are well known. Melamine-based foams are currently manufactured by BASF, located in Ludwigshafen, Germany, under the BASOTECT® brand name. Principles for production of melamine-based foam are disclosed in EP-B 071,671, published by Mahnke et al. on December 17, 1979. According to Mahnke et al. document, they are produced by foaming an aqueous solution or dispersion of a melamine-formaldehyde condensation product which comprises an emulsifier (e.g., metal alkyl sulfonates and metal alkylaryl sulfonates such as sodium dodecylbenzene sulfonate), an acidic curing agent, and a blowing agent, such as a C5 -C7 hydrocarbon, and curing the melamine-formaldehyde condensate at an elevated temperature. The foams are reported to have the following range of properties:
- a density according to DIN 53 420 between 4 and 80 grams per liter (g/l), corresponding to a range of 0.004 g/cc to 0.08 g/cc (though for purposes of the present invention the density may also range from about 0.006 g/cc to about 0.1 g/cc, or other useful ranges);
- a thermal conductivity according to DIN 52 612 smaller than 0.06 W/m °K;
- a compression hardness according to DIN 53 577 under 60% penetration, divided by the density, yielding a quotient less than 0.3 (N/cm²)/(g/l), and preferably less than 0.2 (N/cm²)/(g/l), whereby after measurement of compression hardness the thickness of the foam recovers to at least 70% and preferably at least 90% of its original thickness;
- an elasticity modulus according to DIN 53 423, divided by the density of the foam, under 0.25 (N/mm²)/(g/l) and preferably under 0.15 (N/mm²)/(g/l);
- a bending path at rupture according to DIN 53 423 greater than 6 mm and preferably greater than 12 mm;
- a tensile strength according to DIN 53 571 of at least 0.07 N/mm² or preferably at least 0.1 N/mm²: and,
- by German Standard Specification DIN 4102 they show at least standard flammability resistance and preferably show low flammability.

U.S. Patent No. 6,503,615, issued to Horii et al. on January 7, 2003, discloses a wiping cleaner made from an open-celled foam such as a melamine-based foam, the wiping cleaner having a density of 5 kg/m³ to 50 kg/m³ in accordance with JIS K 6401, a tensile strength of 0.6 kg/cm² to 1.6 kg/cm² in accordance with JIS K 6301, an elongation at break of 8% to 20% in accordance with JIS K 6301 and a cell number of 80 cells/25 mm to 300 cells/25 mm as measured in accordance with JIS K 6402. Melamine-based foam materials having such mechanical properties may be used within the scope of the present invention.

Related foam materials are disclosed in U.S. Patent No. 3,093,600, issued to Spencer et al. on June 11, 1963. Agents are present to improve the elasticity and tear strength of the foam material. Melamine-based foam materials are also disclosed in British Patent No. GB 1,443,024, issued to Russo et al. on July 21, 1976.

A foam material for use in the present invention may be heat compressed to modify its mechanical properties, as described in U.S. Patent No. 6,608,118, issued to Kosaka et al. on August 19, 2003.

Brittle foam materials may be made, as described in German publication DE-AS 12 97 331, from phenolic components, urea-based components; or melamine-based components, in aqueous solution with a blowing agent and a hardening catalyst.

The brittle foam material may comprise organic or inorganic filler particles, such as from about 5% to about 30% by weight of a particulate material. Exemplary particulate materials may include clays such as kaolin, talc, calcium oxide, calcium carbonate, silica, alumina, zeolites, carbides, quartz, and the like. The fillers may also be fibrous materials, such as wood fibers, papermaking fibers, coconut fibers, milkweed fibers, flax, kenaf, sisal, bagasse, and the like. The filler particles or fibers added to the foam material may be heterogeneously distributed or may be distributed homogeneously.

The foam material or a portion thereof may also be impregnated with a material to reinforce or harden the foam material, if desired, such as impregnation with water glass or other silicate compounds, as disclosed in U.S. Patent No. 4,125,664, issued to Giesemann on November 14, 1978. Adhesive materials, hot melts, cleaning agents, bleaching agents (e.g., peroxides), antimicrobials, and other additives may be impregnated in the foam material.

The foam layer may be rectangular in plan view, but may have any other shape, such as semicircles, circles, ovals, diamonds, sinusoidal shapes, dog bone shapes, and the like. The foam layer need not be planar, but may be molded or shaped into three-dimensional topographies for aesthetic or functional purposes. For example, melamine-based foam material may be thermally molded according to the process discussed in U.S. Patent No. 6,608,118, issued to Kosaka et al. on August 19, 2003. The Kosaka et al. document, discussed above, discloses molding the foam at 210 to 350 C (or, more particularly, from 230°C to 280°C or from 240°C to 270°C) for 3 minutes or longer to cause plastic deformation under load, wherein the foam is compressed to a thickness of about 1/1.2 to about 1/12 the original thickness, or from about 1/1.5 to about 1/7 of the original thickness. The molded melamine foams can be joined to a urethane sponge layer to form a composite material, according to the Kosaka et al. document.

As described by Kosaka et al. document, the melamine-based foam may be produced by blending major starting materials of melamine and formaldehyde, or a precursor thereof, with a blowing agent, a catalyst and an emulsifier, injecting the resultant mixture into a mold, and applying or generating heat (e.g., by irradiation or electromagnetic energy) to cause foaming and curing. The molar ratio of melamine to formaldehyde (i.e., melamine:formaldehyde) for producing the precursor is, according to the Kosaka et al. reference, preferably 1:1.5 to 1:4, or more particularly 1:2 to 1:3. 5. The number average molecular weight of the precursor may be from about 200 to about 1,000 or from about 200 to about 400. Formalin, an aqueous solution of formaldehyde, may be used as a formaldehyde source.

Melamine is also known by the chemical name 2,4,6-triamino-1,3,5-triazine. As other monomers corresponding to melamine, there may be used C1-5 alkylsubstituted melamines such as methylolmelamine, methylmethylolmelamine and methylbutylolmelamine, urea, urethane, carbonic acid amides, dicyandiamide, guanidine, sulfurylamides, sulfonic acid amides, aliphatic amines, phenols and the derivatives thereof. As aldehydes, there may be used acetaldehyde, trimethylol acetaldehyde, acrolein, benzaldehyde, furfurol, glyoxal, phthalaldehyde, terephthalaldehyde, and the like.

As the blowing agent, there may be used pentane, trichlorofluoromethane, trichlorotrifluoroethane, and the like. As the catalyst, by way of example, formic acid may be used and, as the emulsifier, anionic surfactants such as sodium sulfonate may be used.

Other useful methods for producing melamine-based foam materials are disclosed in U.S. Patent No. 5,413,853, issued to Imashiro et al. on May 9, 1995. According to Imashiro et al. document, a melamine resin foam of the present invention may be obtained by coating a hydrophobic component on a known melamine-formaldehyde resin foam body obtained by foaming a resin composition composed mainly of a melamine-formaldehyde condensate and a blowing agent. The components used in the present melamine resin foam material may therefore be the same as those conventionally used in production of melamine-formaldehyde resins or their foams, except for the hydrophobic component.

As an example, the Imashiro et al. document discloses a melamine-formaldehyde condensate obtained by mixing melamine, formalin and paraformaldehyde and reacting them in the presence of an alkali catalyst with heating. The mixing ratio of melamine and formaldehyde can be, for example, 1:3 in terms of molar ratio.

The melamine-formaldehyde condensate may have a viscosity of about 1,000-100,000 cP, more specifically 5,000-15,000 cP and may have a pH of 8-9.

As the blowing agent, a straight- chain alkyl hydrocarbon such as pentane or hexane is disclosed.

In order to obtain a homogeneous foam material, the resin composition composed mainly of a melamine-formaldehyde condensate and a blowing agent may contain an emulsifier. Such an emulsifier may include, for example, metal alkylsulfonates and metal alkylarylsulfonates.

The resin composition may further contain a curing agent in order to cure the foamed resin composition. Such a curing agent may include, for example, acidic curing agents such as formic acid, hydrochloric acid, sulfuric acid and oxalic-acid.

The foam material disclosed by Imashiro et al. document may be obtained by adding as necessary an emulsifier, a curing agent and further a filler, etc. to the resin composition composed mainly of a melamine-formaldehyde condensate and a blowing agent, heat-treating the resulting mixture at a temperature equal to or higher than the boiling point of the blowing agent to give rise to foaming, and curing the resulting foam material.

In another embodiment of the present invention, the foam material may comprise a melamine-based foam material having an isocyanate component (isocyanate-based polymers are generally understood to include polyurethanes, polyureas, polyisocyanurates and mixtures thereof). Such foam materials may be made according to U.S. Patent No. 5,436,278, issued to Imashiro et al. on July 25, 1995, which discloses a process for producing a melamine resin foam material comprising a melamine/formaldehyde condensate, a blowing agent and an isocyanate. One embodiment of the present invention includes the production of a melamine resin foam material obtained by reacting melamine and formaldehyde in the presence of a silane coupling agent. The isocyanate component used in U,S. Patent No. 5,436,278 document may be exemplified by CR 200 (a trademark of polymeric-4, 4'-diphenylmethanediisocyanate, produced by Mitsui Toatsu Chemicals, Inc.) and Sumidur E211, E212 and L (trademarks of MDI type prepolymers, produced by Sumitomo Bayer Urethane Co., Ltd). One example therein comprises 100 parts by weight of melamine/formaldehyde condensate (76% concentration), 6.3 parts sodium dodecylbenzenesulfonate (30% concentration), 7.6 parts pentane, 9.5 parts ammonium chloride, 2.7 parts formic acid, and 7.6 parts CR 200. A mixture of these components was placed in a mold and foamed at 100°C, yielding a material with a density of 26.8 kg/m³ (0.0268 g/cm³), a compression stress of 0.23 kgf/cm², and a compression strain of 2.7%. In general, the melamine-based foam materials discussed in U.S. Patent No. 5,436,278 document typically had a density of 25 kg/m³ - 100 kg/m³, a compression strain by JIS K 7220 of 2.7% - 4.2% (this is said to be improved by about 40% - 130% over the 1.9% value of conventional fragile melamine foam materials), and a thermal conductivity measured between 10°C to 55°C of 0.005 kcal/m-h-°C. or less (this is far smaller than 0.01 kcal/m-h-°C. which is said to be the value of conventional fragile foam materials). Other foam materials comprising melamine and isocyanates are disclosed in the World Patent Application No. WO 99/23160, published by Sufi on May 14, 1999, the U.S. equivalent of which is U.S. Patent Application Serial No.98/23864.

In another embodiment of the present invention, a melamine-based foam material may be used that is produced according to the World Patent Application No. WO 0/226872, published by Baumgartl et al. on April 4, 2002. Such foam materials have been tempered at elevated temperature to improve their suitability for use as absorbent articles in proximity to the human body. During or after the tempering process, further treatment with at least one polymer is disclosed, the polymer containing primary and/or secondary amino groups and having a molar mass of at least 300, although this polymer treatment may be skipped, if desired, when the foam materials discussed in the WO 0/226872 document are applied to the present invention. Such foam materials may have a specific surface area determined by BET of at least 0.5 m²/g. Exemplary phenolic foam materials include the dry floral foam materials made by Oasis Floral Products, located in Kent, Ohio, as well as the water-absorbent open-celled brittle phenolic foam materials manufactured by Aspac Floral Foam Company Ltd., located in Kowloon, HongKong, partially described at http://www.aspachk.com/v9/asDac/why aspac.html. Open-cell phenolic foam materials may be made from the phenolic resins of PA Resins, located in Malmö, Sweden, combined with suitable hardeners (e.g., an organic sulfonic acid) and emulsifiers with a blowing agent such as pentane. Phenolic resins may include resole resins or novolac resins, for example, such as the Bakelite® Resin 1743 PS from (Bakelite AG, located in Iserlohn-Letmathe, Germany, which is used for floral foam materials.

### Self-attachment

In several useful embodiments of the present invention, a self-attachment material is provided that comprises both a foam layer and a landing zone disposed on opposing sides of the self-attachment material (e.g., a first surface and a second surface that are integrally joined prior to attachment of the two surfaces with the foam attachment system of the present invention). In one embodiment of the present invention, the self-attachment material is a laminate of a foam layer and a landing layer such as a fibrous loop layer. The foam layer may be provided with free-standing struts rising from an exposed first outer surface of the foam layer. The landing layer serves to provide a second outer surface opposite the first outer surface. When the foam layer (the first outer surface) of the self-attachment material is brought into contact with the landing layer (the second outer surface) of the self-attachment material, effective attachment is possible.

The laminate of the foam layer and the landing layer may be produced by any known means, such as by adhesive bonding, ultrasonic bonding, thermal bonding, hydroentanglement, needling, laser bonding, and fastening by the use of mechanical fasteners such as conventional hook and loop materials. While the foam layer may be joined to the landing layer by engagement of free-standing struts into loops or holes of the landing layer alone, in other embodiments of the present invention, another attachment means may be used to provide greater z-direction bonding strength or peel resistance such that the laminate will not readily come apart under peel forces or other lifting forces (e.g., z-direction forces).

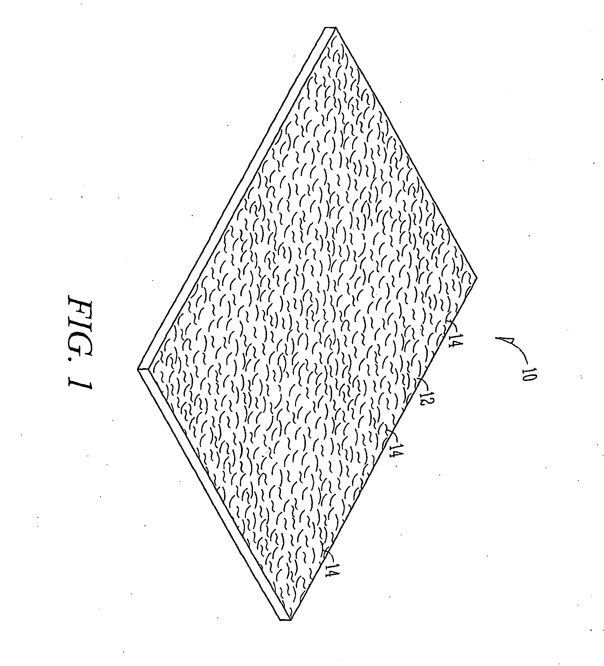

FIG. 1 illustrates a nonwoven fabric 10 that includes a first web 12. The first web 12 is formed of extruded strands 14 that may include an auto-adhesive material.

As used herein, nonwoven fabric refers to a web of material that has been formed without use of weaving processes that typically produce a structure of individual strands which are interwoven in a repeating manner. The nonwoven fabric may be formed by a variety of processes (e.g. meltblowing, spunbonding, film aperturing and staple fiber carding).

Although only a portion of the first web 12 is shown in FIG. 1, it should be noted that the first web 12 may be any size or shape. In addition, the first web 12 may be a variety of different thickness depending on the application where the nonwoven fabric 10 is used. The extruded strands 14 may be formed through any extrusion process that is known now or discovered in the future (e.g., meltblowing).

As used herein, the term "auto-adhesive" refers to self-adhesive properties of a material. An auto-adhesive is substantially non-adhesive with respect to many other materials. Some auto-adhesives may be repeatedly adhered together and separated at service (e.g., room) temperature.

In some embodiments, the auto-adhesive material may be a polymeric material that includes thermoplastic elastomers. As an example, the thermoplastic elastomers may have molecules that include sequential arrangements of unique combinations of monomer units. The thermoplastic elastomers should have relatively stable auto-adhesive properties and be substantially non-adhesive with respect to other materials.

In addition, the auto-adhesive material may include a thermoplastic elastomer that has physical cross-links which restrict the elastomer mobility (i.e., flow). Restricting the elastomeric mobility may promote the auto-adhesive properties of a thermoplastic elastomer.

Some example thermoplastic elastomers that may be used in the auto-adhesive material include multiblock copolymers of radial, triblock and diblock structures including non-rubbery segments of mono- and polycyclic aromatic hydrocarbons, and more particularly, mono- and polycyclic arenes. As examples, mono- and polycyclic arenes may include substituted and unsubstituted poly (vinyl) arenes of monocyclic and bicyclic structure.

In some embodiments, the thermoplastic elastomers may include non-rubbery segments of substituted or unsubstituted monocyclic arenes of sufficient segment molecular weight to assure phase separation at room temperature. As examples, monocyclic arenes may include polystyrene and substituted polystyrenes that have monomer units such as styrene and alkyl substituted styrene (e.g., alpha methylstyrene and 4-methylstyrene). Other examples include substituted or unsubstituted polycyclic arenes that have monomer units (e.g., 2-vinyl naphthalene and 6-ethyl-2-vinyl naphthalene).

It should be noted that the thermoplastic elastomers may also include rubbery segments that are polymer blocks which may be composed of homopolymers of a monomer, or a copolymer that includes two or more monomers selected from aliphatic conjugated diene compounds (e.g., 1,3-butadiene and isoprene). Some example rubbery materials include polyisoprene, polybutadiene and styrene butadiene rubbers. Other example rubbery materials include saturated olefin rubber of either ethylene/butylene or ethylene/propylene copolymers, which may be derived from the corresponding unsaturated polyalkylene moieties (e.g., hydrogenated polybutadiene and polyisoprene).

In addition, the thermoplastic elastomer may be part of a styrenic block copolymer system that includes rubbery segments which may be saturated by hydrogenating unsaturated precursors (e.g., a styrene-butadiene-styrene (SBS) block copolymer that has center or mid-segments which include a mixture of 1,4 and 1,2 isomers). As an example, a -butadiene-styrene (SBS) block copolymer that includes center or mid-segments which have a mixture of 1,4 and 1,2 isomers may be hydrogenated to obtain (i) a styrene-ethylene-butylene-styrene (SEBS) block copolymer; or (ii) a styrene-ethylene-propylene-styrene (SEPS) block copolymer.

In some embodiments, the auto-adhesive material may include a mixture of a polyethylene and a block copolymer. As an example, the auto-adhesive material may include a mixture of one or more block copolymers selected from the group consisting of poly (styrene)-co-poly (ethylene-butylene)-co-poly (styrene) copolymer, poly (styrene)-co-poly (ethylene-butylene) copolymer, and a polyethylene polymer. In some embodiments, the one or more block copolymers may be between about 30 weight percent to about 95 weight percent of the auto-adhesive material, and the polyethylene polymer may be between about 5 weight percent to about 70 weight percent of the auto-adhesive material (wherein all weight percents are based on the total weight amount of the block copolymer and the polyethylene polymer that are present in the auto-adhesive layer).

As used herein, the Peak Load of Auto-adhesive Strength represents a force that is required to separate the nonwoven fabric 10 when it is attached to itself. When the nonwoven fabric 10 is used as an adhesive component, the Peak load of Auto-adhesive Strength should meet the adhesive strength requirement for a particular application. If a nonwoven fabric 10 is used in a fastening system, the Peak Load of Auto-adhesive Strength for the nonwoven fabric 10 needs to be high enough to prevent the fastening system from opening during use. A nonwoven fabric 10 that exhibits too low of a Peak Load of Auto-adhesive Strength may not be suitable for some fastening system applications.

The nonwoven fabric 10 readily bonds to other items that include a similar auto-adhesive material with a strength that is greater than the strength which is generated when the nonwoven fabric 10 is bonded to any other type of material (e.g., a bonding strength that is at least twice as great). As an example, the nonwoven fabric 10 may exhibit a Peak Load of Auto-Adhesive Strength value that is greater than about 100 grams per 2.54 cm (inch) width of the nonwoven fabric 10 (about 118 grams per centimeter width of the layer), and up to about 2000 grams per 2.54 cm (inch) width of the nonwoven fabric 10 (about 787 grams per centimeter width of the layer). The method by which the Peak Load of Auto-Adhesive Strength value for a web is determined is set forth in U.S. Patent No. 6,281,278.

The type of auto-adhesive material that may be used to form the plurality of strands 14 will be selected based on (i) processing parameters; (ii) physical properties; (iii) packaging issues; and (iv) costs (among other factors). The first web 12 should have properties that are required for a particular product and/or process. The physical properties of the auto-adhesive material may be controlled to define properties for the nonwoven fabric 10 such as melting temperature, shear strength, crystallinity, elasticity, hardness, tensile strength, tackiness and heat stability (among other properties).

In some embodiments, the nonwoven fabric 10 may be made by melt spinning thermoplastic materials. This type of nonwoven fabric 10 may be referred to as a spunbond material.

Example methods for making spunbond polymeric materials are described in U.S. Pat. No. 4,692,618 to Dorschner et al., and U.S. Pat. No. 4,340,563 to Appel et al. both of which disclose methods for making spunbond nonwoven webs from thermoplastic materials by extruding the thermoplastic material through a spinneret and drawing the extruded material into filaments with a stream of high velocity air to form a random web on a collecting surface. U.S. Pat. No. 3,692,618 to Dorschner et al. discloses a process wherein bundles of polymeric filaments are drawn with a plurality of eductive guns by very high speed air while U.S. Pat. No. 4,340,563 to Appel et al. discloses a process wherein thermoplastic filaments are drawn through a single wide nozzle by a stream of high velocity air. Some other example melt spinning processes are described in U.S. Pat. No. 3,338,992 to Kinney; U.S. Pat. No. 3,341,394 to Kinney; U.S. Pat. No. 3,502,538 to Levy; U.S. Pat. No. 3,502,763 to Hartmann; U.S. Pat. No. 3,909,009 to Hartmann; U.S. Pat. No. 3,542,615 to Dobo et al., and Canadian Patent Number 803,714 to Harmon.

In some embodiments, desirable physical properties may be incorporated into the nonwoven fabric 10 by forming the strands 14 out of a multicomponent or bicomponent material where at least of one the materials in the bicomponent material is an auto-adhesive material. The auto-adhesive material may be similar to any of the auto-adhesive materials described above.

As used herein, strand refers to an elongated extrudate formed by passing a polymer through a forming orifice (e.g., a die). A strand may include a fiber, which is a discontinuous strand having a definite length, or a filament, which is a continuous strand of material.

Some example methods for making a nonwoven fabric from multicomponent or bicomponent materials are disclosed. U.S. Pat. No. 4,068,036 to Stanistreet, U.S. Pat. No. 3,423,266 to Davies et al., and U.S. Pat. No. 3,595,731 to Davies et al. discloses methods for melt spinning bicomponent filaments to form a nonwoven fabric. The nonwoven fabric 10 may be formed by cutting the meltspun strands into staple fibers, and then forming a bonded carded web, or by laying the continuous bicomponent filaments onto a forming surface and thereafter bonding the web.

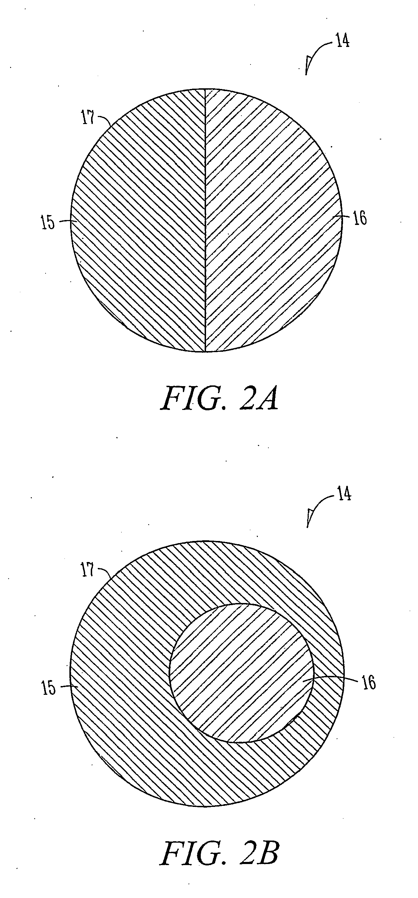

FIGS. 2A-2C illustrate some example forms of bicomponent strands 14 that may be used to form web 12. The strands 14 include a first component 15 and a second component 16 that are arranged in substantially distinct zones across the cross-section of the bicomponent strands 14 and extend along the length of the bicomponent strands 14. The first component 15 of the bicomponent strand includes an auto-adhesive material and constitutes at least a portion of the peripheral surface 17 on the bicomponent strands 14. Since the first component 15 exhibits different properties than the second component 16, the strands 14 may exhibit properties of the first and second components 15, 16.

The first and second components 15, 16 may be arranged in a side-by-side arrangement as shown in FIG. 2A. FIG. 2B shows an eccentric sheath/core arrangement where the second component 16 is the core of the strand 14 and first component 15 is the sheath of the strand 14. It should be noted that the resulting filaments or fibers may exhibit a high level of natural helical crimp in the sheath/core arrangement illustrated in FIG. 2B. In addition, the first and second components 15, 16 may be formed into a concentric sheath/core arrangement as shown in FIG. 2C.

Although the strands 14 are disclosed as bicomponent filaments or fibers, it should be understood that the nonwoven fabric 10 may include strands 14 which have one, two or more components. In addition, the nonwoven fabric 10 may be formed of single component strands that are combined with multicomponent strands. The type of materials that are selected for the first and second components 15, 16 will be based on processing parameters and the physical properties of the material (among other factors).

It should be noted the auto-adhesive material may include additives. In addition, when the strands 14 are formed of a bicomponent (or multicomponent) strands 14, some (or all) of components that form the strands 14 may include additives. As an example, the strands 14 may include pigments, anti-oxidants, stabilizers, surfactants, waxes, flow promoters, plasticizers, nucleating agents and particulates (among other additives). In some embodiments, the additives may be included to promote processing of the strands 14 and/or web 12.

As shown in FIG. 3, the nonwoven fabric 10 may be formed of multiple webs 12, 22, 32. The first web 12 of extruded strands 14 may be similar to first web 12 described above. The first web 12 may be bonded to a second web 22 of extruded strands 14 such that the first and second webs 92, 22 are positioned in laminar surface-to-surface relationship. In addition, the second web 22 may be bonded to a third web 32 such that the second and third webs 22, 32 are positioned in laminar surface-to-surface relationship.

In some embodiments, the second and/or third webs 22, 32 may be a spunbond material while in other embodiments the second and/or third webs 22, 32 may be made by meltblowing techniques. Some example meltblowing techniques are described in U.S. Pat. No. 4,041,203. U.S. Pat. No. 4,041,203 references the following publications on meltblowing techniques: An article entitled "Superfine Thermoplastic Fibers" appearing in INDUSTRIAL & ENGINEERING CHEMISTRY, Vol. 48, No. 8, pp. 1342-1346 which describes work done at the Naval Research Laboratories in Washington, D.C.; Naval Research Laboratory Report 111437, dated Apr. 15, 1954; U.S. Pat. Nos. 3,715,251; 3,704,198; 3,676,242; and 3,595,245; and British Specification No. 1,217,892.

Each of the second and third webs 22, 32 may have substantially the same composition as the first web 12 or have a different composition than the first web 12. In addition, the second and third webs 22, 32 may be formed from single component, bicomponent or multicomponent strands 14.

In some embodiments, the first, second and/or third webs 12, 22, 32 may formed separately and then bonded together (e.g., by thermal point bonding). It should be noted that when the first, second and possibly third web are bonded together, and a common elastomeric polymer is present in the strands 14 that form the first, second and third webs 12, 22, 32, the bonding between the first, second and third webs 12, 22, 32 may be more durable.

In other embodiments, the first, second and third webs 12, 22, 32 may be formed in a continuous process wherein each of the first, second and third webs 12, 22, 32 is formed one on top of the other. Both processes are described in U.S. Pat. No. 4,041,203.

The types of materials that are selected for the extruded strands 14 that make up the first, second and third webs 12, 22, 32 will be based on processing parameters and the desired physical properties of the nonwoven fabric 10 (among other factors). The first, second and third webs 12, 22, 32 may be attached together through any method that is known now or discovered in the future. Although the first, second and third webs 12, 22, 32 are partially shown as webs of the same size, it should be noted that the first, second and third webs 12, 22, 32 may be different sizes and/or shapes. In addition, the first, second and third webs 12, 22, 32 may be the same (or different) thicknesses.

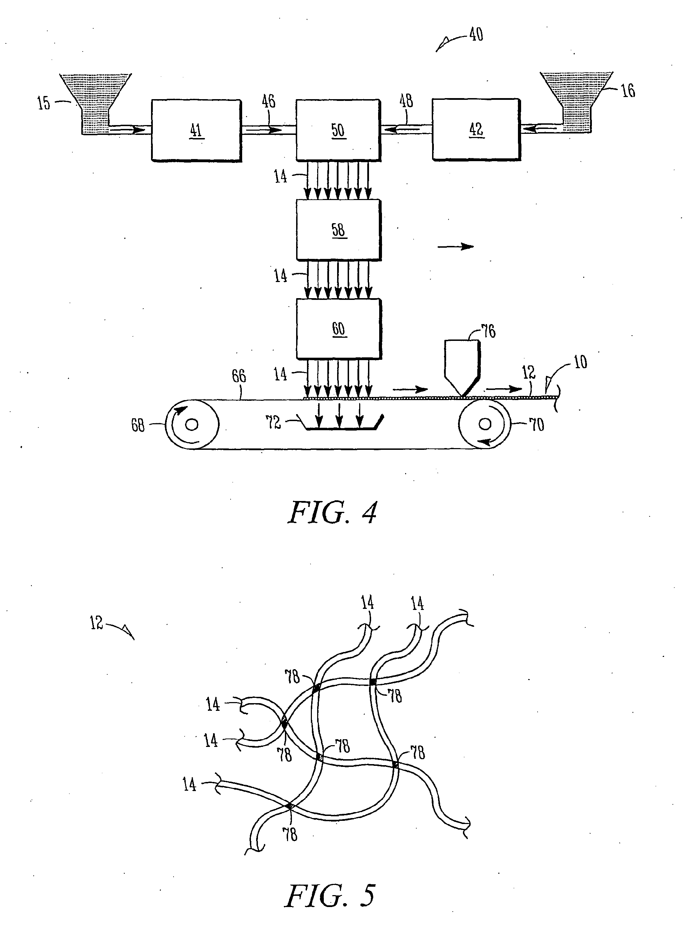

A method of forming a nonwoven fabric 10 will now be described with reference to FIG. 4. The method includes extruding a plurality of strands 14 where at least some of the strands 14 may be formed of an auto-adhesive material. The method further includes routing the plurality of strands 14 toward a moving support 66 and depositing the plurality of strands 14 onto the moving support 66. The method further includes stabilizing the plurality of strands 14 to form a web 12.

FIG. 4 shows an example processing line 40 that is arranged to produce a web 12 that includes a plurality of bicomponent continuous strands 14 (e.g., filaments orfibers). It should be understood that the processing line 40 may be adapted to form a nonwoven fabric 10 that includes one, two or multiple components in each strand 14. In addition, the processing line 40 may be adapted to form a nonwoven fabric 10 that include single component strands 14 in combination with multicomponent strands 14.

In the example embodiment that is illustrated in FIG. 4, the first and second components 15, 16 may be separately co-extruded in two different extruders 41, 42. It should be noted that the first and second extruders 41, 42 may be any extruder that is known now or discovered in the future.

In some embodiments, the first and second components 15, 16 are in the form of solid resin pellets (or particles) that are heated above their melting temperature and advanced along a path (e.g., by a rotating auger). The first component 15 is routed through one conduit 46 while the second component 16 is simultaneously routed through another conduit 48.

Both flow streams are directed into a spin pack 50 that initially forms the strands 14. As an example, the spin pack 50 may include a plate that has a plurality of holes or openings through which the extruded material flows. The number of openings per square inch in the spin pack 50 may range from about 5 to about 500 openings per square inch. The size of each opening in the spin pack may vary from about 0.1 millimeter (mm) to about 2.0 mm in diameter. It should be noted that the openings in the spin pack 50 may have a circular cross-section, or have a bilobal, trilobal, square, triangular, rectangular or oval cross-section depending on the properties that are desired for the nonwoven fabric 10.

In the example embodiment that is illustrated in FIG. 4, the first and second components 15, 16 may be directed into the spin pack 50 and then routed through the spin pack 50 in such a manner that the second component 16 forms a core while the first component 15 forms a sheath which surrounds the core. As discussed above with regard to FIGS. 2A-2C, the bicomponent strands 14 may have a side by side configuration or a core/sheath design (among other possible configurations).

One bicomponent strand 14 will be formed for each opening formed in the plate within the spin pack 50. Each of the plurality of strands 14 simultaneously exits the spin pack 50 at a first speed. The initial diameter of each bicomponent strand 14 will be dictated by the size of the openings that are in the plate of the spin pack 50.

In some embodiments, the plurality of strands 14 are routed downwardly through a quench chamber 58 to form a plurality of cooled strands 14. It should be noted that directing the strands 14 downward allows gravity to assist in moving the strands 14. In addition, the downward movement may aid in keeping the stands 14 separated from one another.

The strands 14 are contacted by one or more streams of air as the strands move into the quench chamber 58. The velocity of the incoming air may be maintained or adjusted so that the strands 14 are efficiently cooled.

The plurality of strands are then routed to a draw unit 60 that may be located below the quenching chamber 50 so as to again take advantage of gravity. As used herein, drawing involves subjecting the cooled strands 14 to pressurized air that draws (i.e., pulls) the molten strands 14 which are exiting the spin pack 50 downward.

The downward force that is generated by the pressurized air in the draw unit 60 causes the molten strands 14 to be lengthened and elongated. The amount that the diameter of the strands 14 is reduced depends upon several factors including (i) the number of molten strands 14 that are drawn; (ii) the distance over which the strands 14 are drawn; (iii) the pressure and temperature of the air that is used to draw the strands 14; and (iv) the spin line tension (among other factors).

The cooled strands 14 are pulled within the draw unit 60 at a speed that is faster than the speed at which the continuous molten strands 14 exit the spin pack 50. The change in speed causes the molten strands to be lengthened and reduced in cross-sectional area. The cooled strands 14 may be completely solid upon exiting the draw unit 60.

The solid strands 14 are deposited onto a moving support 66 after exiting the draw unit 60. As an example, the moving support 66 may be a continuous forming wire or belt that is driven by a drive roll 68 and revolves about a guide roll 70.

The moving support 66 may be constructed as a fine, medium or coarse mesh that has no openings or a plurality of openings. As examples, the moving support 66 may have a configuration that is similar to a standard window screen, or the moving support 66 may be tightly woven to resemble a wire that is commonly used by the paper industry in the formation of paper. A vacuum chamber 72 may be positioned below the moving support 66 to facilitate accumulation of the strands 14 onto the moving support 66.

In some embodiments, the strands 14 accumulate on the moving support 66 in a random orientation such that the accumulation of strands 14 at this point does not include any melt points or bonds that would stabilize the strands 14 into a web. The thickness and basis weight of the strands 14 is established in part by (i) the speed of the moving support 66; (ii) the number and diameter of the strands 14 that are deposited onto the moving support 66; and (iii) the speed at which the strands 14 are being deposited onto the moving support 66.

Depending on the type of processing line 40, the moving support 66 may route the plurality of strands 14 under a hot air knife 76 that directs one or more streams of hot air onto the plurality of strands 14. The hot air needs to be of sufficient temperature to melt some of the strands 14 at points where the strands 14 contact, intersect or overlap other strands 14.

As shown in FIG. 5, the strands 14 adhere to adjacent strands 14 at melt points 78 to form a stabilized web 12. The number of melt points 78 that form the web 12 is determined by a number of factors including: (i) the speed of the moving support 66; (ii) the temperature of the hot air; (iii) the types of material that are in the strands 14; and (iv) the degree to which the strands 14 are entangled (among other factors).

In some embodiments, the web 12 may be routed through a nip that is formed by a bond roll (not shown) and an anvil roll (not shown) which are heated to an elevated temperature. As an example, the bond roll may contain one or more protuberances that extend outward from the outer circumference of the bond roll. The protuberances may be sized and shaped to create a plurality of bonds in the web 12 as the web 12 passes through the bond roll and the anvil roll. Once the web 12 has bonds formed therein, the web 12 becomes a bonded web 12.

The exact number and location of the bonds in the bonded web 12 is determined by the position and configuration of the protuberances that are on the outer circumference of the bond roll. As an example, at least one bond per square inch may be formed in the bonded web 12, although embodiments are contemplated where the percent bonded area varies. As an example, the percent bonded area may be from about 10% to about 30% of the total area of the web 12.

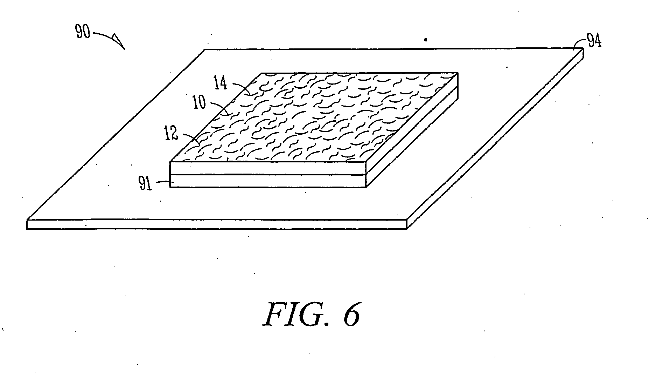

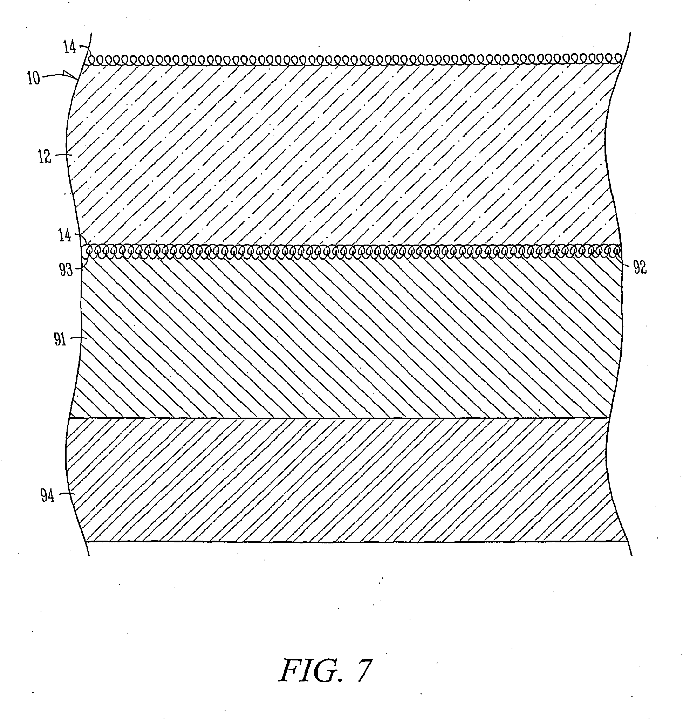

FIGS. 6 and 7 depict a fastening system 90. The fastening system 90 includes a nonwoven fabric 10 that has a web 12 which is formed of a plurality of extruded strands 14 where at least some of the strands 14 may include an auto-adhesive material. The fastening system 90 includes a foam layer 91 that has a surface 92 (see FIG. 7) which is formed of a plurality of free-stranding struts 93. At lease a portion of the surface 92 of the foam layer 91 include a surface modifier (not shown). The free-standing struts 93 are adapted to engage at least a portion of the plurality of strands 14.

It should be noted that the nonwoven fabric 10 may be similar to any of the nonwoven fabrics 10 that are described above. In addition, the foam layer 91 may be similar to any of the foam layers that are described in U.S. patent Publication No. 2006-0069380A1 (application serial no. 10/956613) filed, September, 30, 2004 and European Patent 0235949A1. As an example, the foam layer 91 may be an open cell foam.

The surface modifier that is used on the surface 92 of the foam layer 91 may be similar to any of the auto-adhesive materials described above Further the surface modifier that is used on the surface 92 of the foam layer 91 may be a low-tack adhesive or polymer wax. The types of surface modifier that is selected for the foam layer 91 that makes up the fastening system 90 will be based on processing parameters and the desired physical properties of the fastening system 90 (among other factors).

The surface modifier used on the surface 92 of the foam layer 91 may be applied utilizing numerous methods, for example spray nozzles, glue guns, bead applicators, extruders, gravure printing, flexographic printing, ink-jet printing, coating, and the like. The surface modifier may be, but need not be, uniformly applied to surface 92 of the foam layer 91, and may be applied selectively in regions. The surface modifier may also be applied in a pattern or in a substantially random distribution.

The surface modifier used on the surface 92 of the foam layer 91 at any add-on as may be required, however to provide beneficial cost, the add-on may be less than 100 gsm, alternatively less than 50 gsm, alternatively less than 30 gsm or alternatively less than 25 gsm. To provide the desired benefit, the surface modifier may be used on the surface 92 of the foam layer 91 at an add-of of greater than 1 gsm, alternatively greater than 5 gsm, alternatively greater than 10 gsm or alternatively greater than 15 gsm.

The surface modifier may improve the bonding of the foam layer 91 to the strands 14 of the web 12 as compared to a foam layer 91 which does not have a surface modifier on the surface 92. A strength of a bond between the web 12 and a portion of the foam layer 91 including the surface modifier may be greater than 1.5 times, alternatively greater than 2.5 times or alternatively greater than 2.0 times a strength of a bond between the web 12 and a portion of the foam layer 91 not including the surface modifier. The strength of the bond may be measured by the peak shear load, peak peal, or other suitable test method.

The surface modifier may improve the bonding of the foam layer 91 by a number of mechanisms. For example the surface modifier may improve the attachment by stiffening the surface 92 of the foam layer 91 thereby improving the mechanical interlocking between the surface 92 and the nonwoven fabric 10. When the primary mechanism for improvement is improved mechanical interlocking, there may be minimal decrease in peel and shear strength when the surface modifier/ foam layer 91 surface 92 has been contaminated. For example when the foam layer 91 surface 92 is contaminated with water, for example in the "moist" test method as described below,
the "moist" attachment of the foam layer 91 including the surface modifier and the nonwoven fabric 10 may be greater than 90%, alternatively greater than 80% or alternatively greater than 60% of a "dry" attachment of the foam layer 91 including the surface modifier and the nonwoven fabric10 as tested by the moist shear or moist peel test as described below.

In some embodiments, the surface modifier that is used on the surface 92 of the foam layer 91 may be similar or identical to an auto-adhesive that may be included on some of the plurality of strands 14.

In some embodiments, at least some of the plurality of strands 14 may include an auto-adhesive material that may form auto-adhesive loops that engage the auto-adhesive free-standing struts 93 of the foam layer 91. In addition, at least a portion of some of the auto-adhesive free-standing struts 93 may form auto-adhesive hooks such that the auto-adhesive hooks are adapted to engage the auto-adhesive loops on the web 12.

It should be noted that the extent to which the strands 14 form loops and the free-standing struts 93 form hooks will depend in part on how the respective nonwoven fabric 10 and foam layer 91 are fabricated. As an example, the free-standing struts 93 may have diameters of about 500 microns or less.

In some embodiments, the foam layer 91 may be reinforced by attaching a support 94 to the foam layer 91. The support 94 may be attached to the foam layer 91 by any means (e.g., adhesive lamination of the support 94 to the foam layer 91 or formation of the foam layer 91 on the support 94). As an example, the support 94 may be dipped into a liquid that is cured to form the foam layer 91. U.S. Patent No. 6,613,113, issued to Minick et al. on September 2, 2003 describes such a process.

Adding the support 94 to the foam layer 91 may improve strength and/or flexibility of the foam layer 91. Improving the strength and flexibility of the foam layer 91 may increase the number of applications where the fastening system 90 may be used.

In some embodiments, the free-standing struts 93 of the foam layer 91 may be treated to have increased surface roughness which may facilitate attachment of the free-standing struts 93 to the nonwoven fabric 10. As an example, the free-standing struts 93 may be roughened by attaching particles to them (e.g., microspheres, mineral filler, etc.).

In other embodiments, the free-standing struts 93 may be etched or otherwise treated (e.g., by chemical attack, laser ablation, electron beam treatment, etc.) to remove portions of the surface material in individual free-standing struts 93. U.S. Patent No. 3,922,455, issued to Brumlik et al. on November 25, 1975 describes some examples of textured elements that may correspond to modified free-standing struts 93.

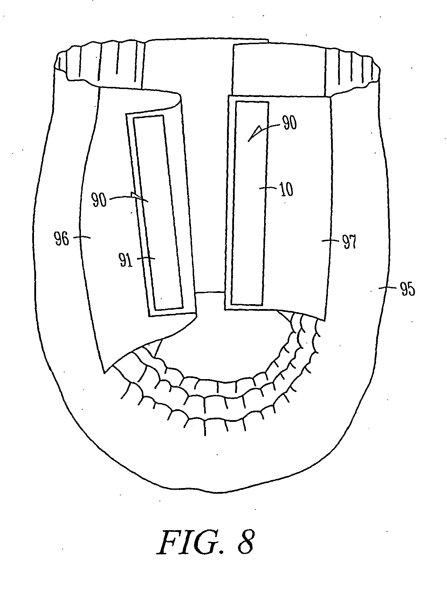

FIG. 8 illustrates an example disposable absorbent article 95 (shown as a training pant) that may include any of fastening systems 90 described herein. The illustrated example absorbent article 95 is similar to the training pant disclosed in U.S. Patent No. 6,562,167, issued to Coenen et al. on May 13, 2003.

The example absorbent article 95 is illustrated in a partially fastened mode in FIG. 8. In the illustrated example embodiment, the foam layer 91 of the fastening system 90 is joined to front side panels 96 on the training pant 95 and a portion of the nonwoven fabric 10 is attached to rear panels 97 on the training pant 95. The fastening system 90 secures the training pant 95 about the waist of a wearer by engaging the nonwoven fabric 10 with the foam layer 91.

The fastening system 90 of the present invention may be useful in a variety of other applications. As examples, the fastening system 90 may incorporated into other products such as adult incontinent products, bed pads, other catamenial devices, sanitary napkins, tampons, wipes, bibs, wound dressings, surgical capes or drapes, soiled garment bags, garbage bags, storage bags and product packaging. The fastening system 90 may be especially well suited to diaper-related applications because surface modifier or the auto-adhesive material in the nonwoven fabric 10 is not readily contaminated with many of the materials that are commonly present in diaper changing environments (e.g., baby lotions, oils and powders).

The fastening system 90 may be secured to diapers (or other products) using thermal bonding and/or adhesives (among other techniques). As an example, one section of the fastening system 90 may be secured to one portion of a diaper such that the section is designed to engage another section of the fastening system 90 on another portion of the diaper.

The fastening system 90 may also be decorative in color and/or shape depending on consumer appeal. There are also embodiments that are contemplated where the fastening system 90 has an unobtrusive product form such that the fastening system 90 does not interfere with the aesthetics of the products where the fastening system 90 is located.

Now specific attention will be given to physical samples which were created to demonstrate the present invention.

### Representative Example 1

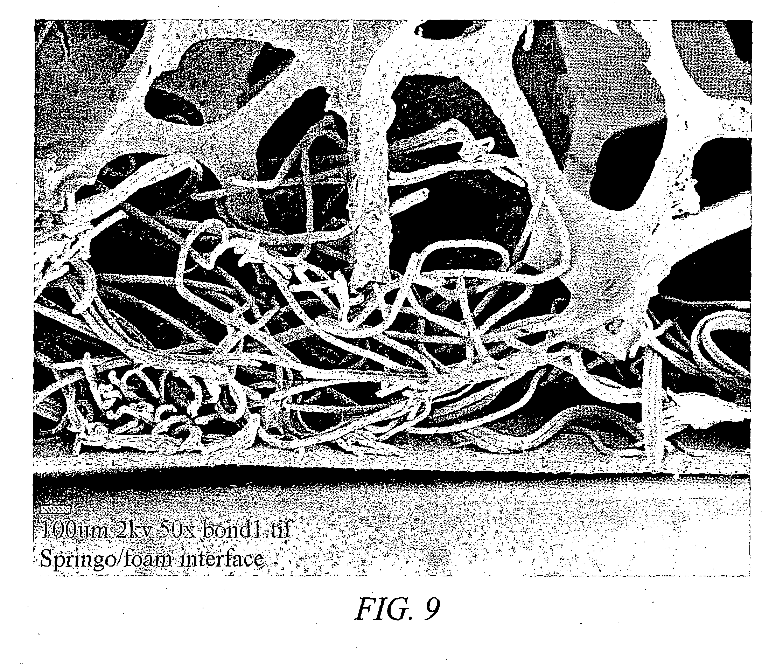

An SEM photomicrograph was obtained showing a representative reticulated foam engaged with a representative nonwoven fabric, FIG. 9. Specifically, Z65CLY, a fully reticulated foam produced by Foamex International located in Eddystone, PA, having a fully reticulated structure with all membranes between foam cells removed and a thickness of 3mm and density of 65 pores per 2.54 cm (inch) was engaged in an elastic nonwoven fabric as described in U.S. Patent Publication 20040110442 filed August 30, 2002 and U.S. Patent Publication No. 2006-0135021 A1 (Application 11/017984) filed December 20, 2004.

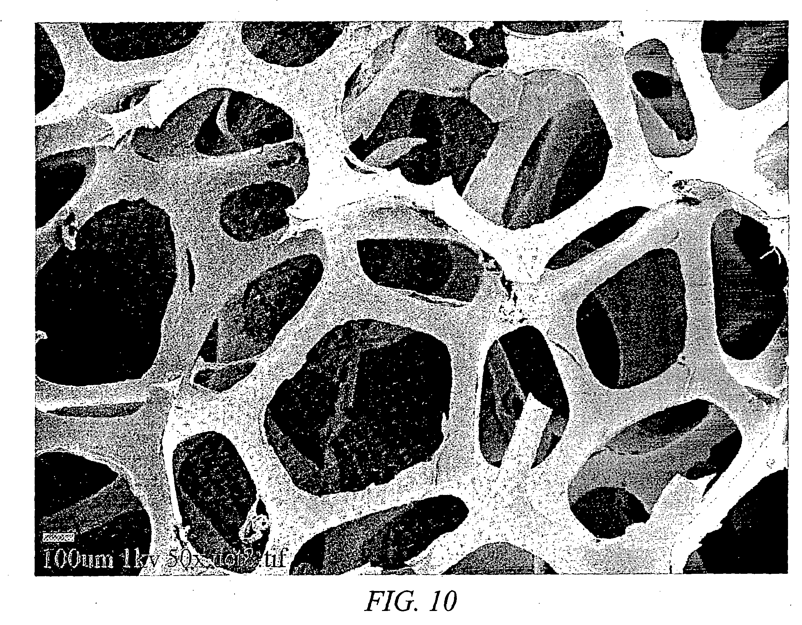

Examination at low magnification with reflected light and transmitted light microscopy of both the outer surfaces and of a cross-section of the foam material cut in half show that the foam material is a substantially uniform block of semirigid foam material with an open cell structure. For example, FIG. 9 was taken at 50X magnification in transmitted light showing a razor-cut cross-sectional surface of the Z65CLY foam which is engaged in an elastic nonwoven fabric. The foam material was cut in half through its center after engagement with the nonwoven fabric. All surfaces of the foam material, inside and outside, appear substantially as shown in FIG. 10, showing a network of interconnected filaments serving as struts in an open-celled foam network that appeared to be substantially uniform throughout. Further, as shown in FIG. 9 the free-standing struts on the surface of the foam can releasably attach to the non-woven by means of catching fibers under the struts, or struts of the foam latching underneath fibers or fiber clusters. Foam material samples were prepared for SEM analysis by cutting out a cube 1.27 cm (½") on a side with a razor blade. Thinner segments of the foam material were cut from the cube and mounted onto a 2.54 cm (1") diameter flat disc holder with double-stick tape. The mounted foam material samples were metallized with gold using a vacuum sputter coater to approximately 250 angstroms thickness. SEM analysis was performed with a JSM-840 electron microscope available from Jeol USA Inc., located in Peabody, Maine, with an accelerating voltage of 5 kV, a beam current of 300 picoAmps, a working distance of 36 to 12 millimeters, and magnification of 30X to 15,000X.

### Representative Examples 2A, 2B, 2C, 2D, 2E

The Z65CLY foam was coated with a surface modifier, specifically H9078-01 from Bostic, Inc. located in Wauwatosa. The H9078-01 has an application temperature range form ∼121 °C (∼250°F) to ∼1492C (∼300°F). The H9087-01 is tacky at elevated temperatures, but becomes essentially non-tacky as it cools to room temperature. The Z65CLY foam was coated with the H9078-01 with a meltblown adhesive applicator utilizing the following conditions: melt tank temperature 149°C (300°F); die temperature 143°C (290°F), air temperature 185°C (365°F); nip pressure 4378 n/m (25 pli), air pressure 117.2 kPa (17 psig); line speed 9.15 m/min (30 ft/min): forming height 4.45 cm (1.75 inches); and open time 0.2 sec. Samples of coated foam were covered with release paper following the coating procedure to prevent roll blocking and protect the coat. Five different samples were produced that different in the add-on levels of the coat.
Sample 2A - 0 gsm add-on
Sample 2B - 5 gsm add-on
Sample 2C - 10 gsm add-on
Sample 2D - 15 gsm add-on
Sample 2E - 20 gsm add-on

### SEM Photomicrography

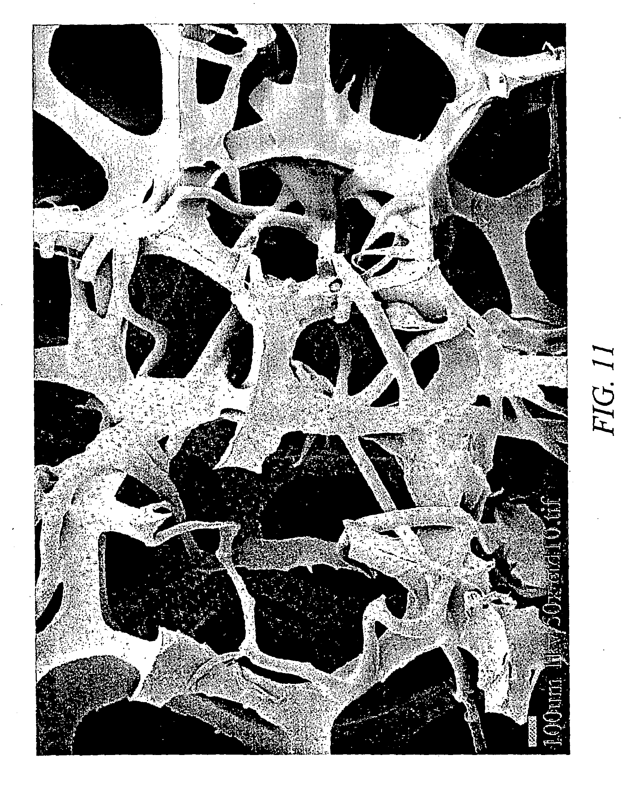

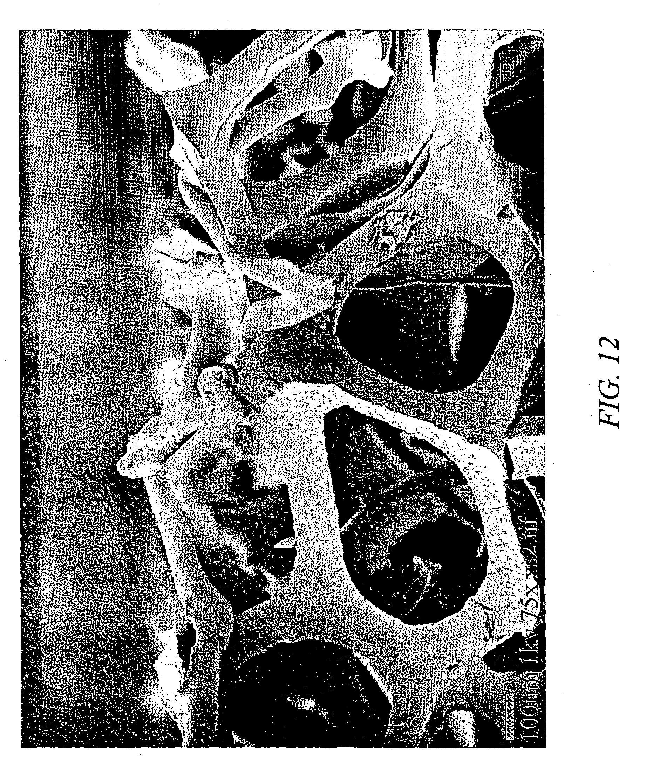

FIG. 10 is an SEM photomicrograph at 50X magnification of the surface of sample 2A (0 gsm add-on). FIG. 11 is an SEM photomicrograph at 50X magnification of the surface of sample 2C (10 gsm add-on). FIG. 12 is an SEM photomicrograph at 75X magnification of a razor-cut cross-sectional surface of sample 2C (10 gsm add-on). The SEM images show that the H9078-01 coating appears either as strings or as irregular lumps on the foam cells. The H9078-01 coating is often seen to drape over the strut edges or wrap aground the struts. The H9078-01 coating appears to be confined mostly to the surface or near the surface to a depth of about one or two cells. This is most evident in the cross-sectional view, FIG. 11.

It should be noted that the H9078-01 coating does not fill up the open cells or totally cover or block the surface. Therefore the number of free-standing struts capable of engagement remains almost unchanged. Further, there remains a significant amount of open space (foam cell holes) that provide for the breathability of the coated foam material. This distinguishes it favorably from conventional hook material that is generally non-breathable.

### Curved Shear Attachment Strength

The curved shear strength of the bonding of Samples 2A, 2B, 2C, 2D and 2E with a model nonwoven fabric were measured to assess how the coating procedure affected the ability of the foam layer to attach to fibrous landing layers. The model nonwoven fabric was an SBL material, specifically the waistband material of Huggies® Convertibles Diapers (SBL) and described in U.S. Patent U.S. Patent 4,720,495 issued January 19, 1988 to Taylor et al. More specifically the SBL material was created with two 0.4 osy polypropylene spundbond facings and a 44 gsm (1.298 osy) Kraton G2760 core. Further, the SBL had a 232% unreferenced stretch-to-stop. Results are shown in Table 1.

### Curled Shear Attachment Strength Test Method

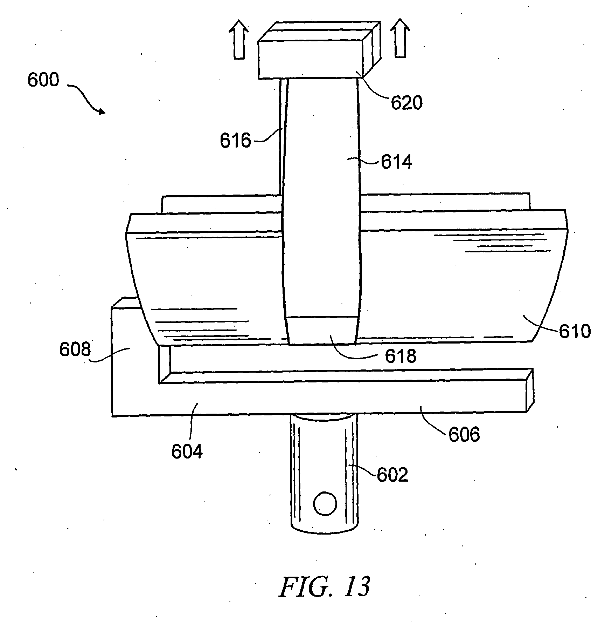

The shear attachment strength of attachment of foam layers to landing layers of the present invention was obtained using a universal testing machine, an MTS Alliance RT/1 testing machine (commercially available from the MTS Systems Corp., located at Eden Prairie, Minnesota) running with TestWorks® 4 software, version 4.04c, with a 100 N load cell. For the test procedure, an upper clamp was used with rubber-lined jaws that are pneumatically loaded for good grasping of test samples. Into the lower mount of the test device was placed a special rig as shown in FIG. 13 which provided a curved surface against which an overlapping region of a foam layer and landing layer could be subject to tensile force. In FIG. 13, the test rig 600 comprises a cylindrical base 602 adapted for mounting into the lower mount of the universal testing machine (not shown), joined to a an attachment section 604 comprising a horizontal beam 606 and a vertical beam 608 which is bolted into a curved section 610.

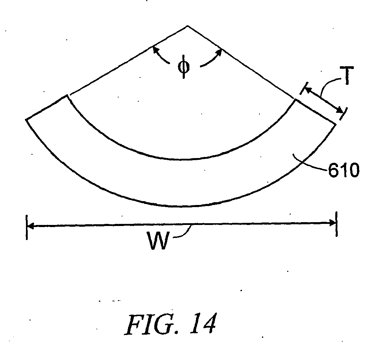

Further details about the geometry of the curved section 610 are shown in the cross-sectional view of FIG. 14, which shows that the curved section 610 represents a circular arc subtending an angle φ of 110 degrees, has a thickness T of 1.27 cm (0.5 inches), and a width W of 11.4 cm (4.5 inches). The length of the curved section 610, the distance it extends into the plane of the paper in FIG. 14 (the left-to-right distance spanned by the curved section 610 in FIG. 13) is 20.3 cm (8 inches). The curved section 610 made of rigid nylitron and has a smooth surface finish (a shape turned finish) of 32 microinches in roughness (a "32 finish") as measured with a Microfinish Comparator (Gar Electroforming, Danbury, Connecticut).

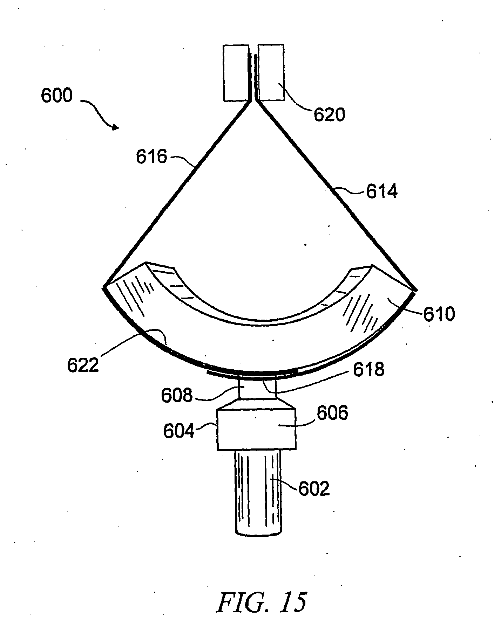

As shown in FIG. 13 and also in a side view in FIG. 15, the curved section 610 is used to hold a length of a two-inch wide foam layer strip 614 and a length of a three-inch wide landing layer strip 616 that overlap and are joined in an attachment zone 618 while the remote ends of the foam layer strip 614 and the landing layer strip 616 are also held in an upper clamp 620 connected to the movable head (not shown) of the universal testing machine (not shown). The joining of the foam layer and landing layer strips 614 and 616, respectively, in the attachment zone 618 is carried out by superposing the laterally centered, aligned foam layer and landing layer strips 614 and 616, respectively, to from an overlap region 612 that was 1 inch long and then applying a load to ensure good contact. Unless otherwise specified, the load was provided by a brass laboratory roller having a mass of 7.0 kilograms, which was slowly rolled over the attachment zone 618 twice (forward and then back), After attaching the foam layer and landing layer strips 614 and 616, respectively, the attachment zone 618 is then centered on the lower portion of the curved section 610 and the ends of the foam layer and landing layer strips 614 and 616, respectively, remote from the attachment zone 618 are then placed in the jaw of the upper clamp 620. The lower surface of the upper clamp 620 is 3 inches above the upper surface of the curved section 610 before the test procedure begins. There is negligible tension yet no significant slack in the foam layer and landing layer strips 614 and 616, respectively, before the test procedure begins.

A measure of the strength of the attachment in the overlap region 612 may be obtained by running the universal test machine as if a tensile test were being carried out and measuring the peak load at failure. The test procedure is executed by moving the upper mount upwards at a crosshead speed of 10 inches per minute until there is failure, which may be failure of the attachment zone 618 or, in some cases, breaking of one of the foam layer and landing layer strips 614 and 616, respectively, elsewhere. The peak load before failure is the attachment strength.

**Table 1 Curved Shear Attachment Strength**

| Sample | Peak Load, gf 1 gram-force = 0.0098N | | | n* | Energy to peak, g*cm | | |
|---|---|---|---|---|---|---|---|
| | Avg. | S. Dev. | %COV | | Avg. | S.Dev. | %COV |
| 2A - SBL | 470 | 87 | 18 | 5 | 839 | 281 | 33 |
| 2B - SBL | 1654 | 179 | 11 | 5 | 11381 | 3224 | 28 |
| 2C - SBL | 1554 | 412 | 27 | 10 | 11797 | 2664 | 23 |
| 2D - SBL | 1939 | 200 | 10 | 5 | 10584 | 3043 | 29 |
| 2E - SBL | 2036 | 213 | 10 | 5 | 19557 | 4875 | 25 |

| | | | | | | | |
|---|---|---|---|---|---|---|---|
| n* - number of specimen tested per sample | | | | | | | |

The testing indicates that the coating resulted in a significant increase in the attachment strength as measured by the peak shear load: 3.5 to 4 times depending on the basis weight of the coating. Further, the attachment strength generally increased with an increase in the basis weight of the coating.

Further curved shear attachment strength testing was conducted on two additional nonwoven fabrics. Again the first nonwoven fabric was the SBL material which forms the back waist band on Huggies® Convertibles diapers (SBL). The second nonwoven fabric was the SBL material with fibers modified through a picking/combing process to have more loft (Modified-SBL). Specifically, the original SBL was subjected to a mechanical modification process that increased the availability for engagement of the fibers in the engaging surface with reticulated foams. The engaging surface of SBL was mechanically modified with a 15 lb. hand roller that had a sheet of Velcro® 85-1065 (commercially available from Velcro USA Inc. of Manchester, NH) hook material wrapped around the outer surface, such that the hooks of the hook material extended away from the roll. The engaging surface of each fibrous non-woven web was treated with this hook-wrapped roller by rolling the wrapped roller over the engaging surface two times back and forth in one direction and two times back and forth in a direction 90 degrees to the first direction.

The third nonwoven fabric was an elastic nonwoven fabric as described in U.S. Patent Publication 2005/0101206 filed August 13, 2004 and U.S. Patent No. 7238423 (Application 11/017984) filed December 20, 2004 (elastic nonwoven). Specifically the elastic nonwoven material has a facing that is 27.1 gsm (0.8 osy) bicomponent sheath/core spunbond comprised of an 80wt% core of Dow EG8185 metallocene polyethylene and 20% sheath of Dow Aspun 6811A polyethylene. The elastic nonwoven has a breathable elastic film that is described in Example 5 (page 15, paragraphs 149 and 150 of US 2005/0101206). The elastic nonwoven is adhesively laminated to a film with Bostic H9375 adhesive. (adhesive is disclosed in example 7, page 16, of US 2005/0101206).

Each of the three nonwoven fabrics (SBL, modified SBL and elastic nonwoven) were bonded with 2A (0 gsm add-on) and 2C (10 gsm add-on) and tested according to the test set forth above. The results are set for the in Table 2.

**Table 2 Curved Shear Attachment Strength**

| Sample | Peak Load, gf 1 gram-force = 0.0098N | | | n* | Energy to peak, g*cm | | |
|---|---|---|---|---|---|---|---|
| | Avg. | S. Dev. | %COV | | Avg. | S.Dev. | %COV |
| 2A - SBL | 470 | 87 | 18 | 5 | 839 | 281 | 33 |
| 2C-SBL | 1554 | 412 | 27 | 10 | 11797 | 2664 | 23 |
| 2A - Modified SBL | 1223 | 185 | 15 | 3 | 5181 | 1589 | 31 |
| 2C - Modified SBL | 2626 | 804 | 31 | 4 | 32212 | 13052 | 41 |
| 2A - Elastic nonwoven | 939 | 95 | 10 | 4 | 2436 | 631 | 26 |
| 2C - Elastic nonwoven | 2571 | 454 | 18 | 4 | 22312 | 7946 | 36 |

| | | | | | | | |
|---|---|---|---|---|---|---|---|
| n* - number of specimen tested per sample | | | | | | | |

### Refastenability

Testing was further conducted to determine the refastenability of the coated Z65CLY foam. Refastenability is required for many disposable garment applications in order to provide more comfort and better fit of the product to the wearer. Refastenability of the coated foam (Sample 2C -10 gsm add-on) and two different nonwoven fabrics (modified SBL and elastic nonwoven) was tested with the results presented in Tables 3 and 4. Two samples were tested per code (x₁, x₂). After the first attachment was measured, the testing apparatus was reset. Then the test material rejoined as described above and the second attachment was measured. This was repeated for the third, fourth and fifth attachment.

**Table 3 Refastenability of Attachment for 2C and Modified SBL Curved Attachment Strength**

| Sample | Peak Load, gf 1 gram-force = 0.0098N | | | | | Energy to peak, g*cm | | | | |
|---|---|---|---|---|---|---|---|---|---|---|
| | X₁ | X₂ | Avg. | S. Dev. | % COV | X₁ | X₂ | Avg. | S. Dev. | % cov |
| 1^{st} attachment | 2588 | 1992 | 2290 | 421.8 | 18.4 | 28569 | 17985 | 23277 | 7484.0 | 32.2 |
| 2^{nd} attachment | 3414 | 2260 | 2837 | 816.5 | 28.8 | 35774 | 20797 | 28286 | 10590.7 | 37.4 |
| 3^{rd} attachment | 3027 | 2112 | 2569 | 646.5 | 25.2 | 27390 | 16214 | 21802 | 7902.3 | 36.2 |
| 4^{th} attachment | 2614 | 2011 | 2312 | 426.5 | 18.4 | 22135 | 14595 | 18365 | 5331.6 | 29.0 |
| 5^{th} attachment | 2003 | 1776 | 1890 | 160.2 | 8.5 | 14631 | 12030 | 13330 | 1838.8 | 13.8 |

**Table 4 Refastenability of Attachment for 2C and Elastic nonwoven Curved Attachment Strength**

| Sample | Peak Load, gf 1 gram-force = 0.0098N | | | | | Energy to peak, g*cm | | | | |
|---|---|---|---|---|---|---|---|---|---|---|
| | X₁ | X₂ | Avg. | S. Dev. | % COV | X₁ | X₂ | Avg. | S. Dev. | % COV |
| 1^{st} attachment | 2872 | 2956 | 2914 | 59.0 | 2.0 | 28203 | 29291 | 28747 | 769.3 | 2.7 |
| 2^{nd} attachment | 3419 | 3417 | 3418 | 1.8 | 0.1 | 32390 | 30166 | 31278 | 1572.6 | 5.0 |
| 3^{rd} attachment | 3201 | 3328 | 3265 | 89.6 | 2.7 | 24488 | 24874 | 24681 | 273.2 | 1.1 |
| 4^{th} attachment | 2402 | 2837 | 2620 | 307.7 | 11.7 | 16124 | 23203 | 19663 | 5005.8 | 25.5 |
| 5^{th} attachment | On the 5^{th} attachment for both x₁ and x₂, the elastic nonwoven broke before the bond broke. | | | | | | | | | |

The results of the testing indicates good refastenability with both nonwoven fabrics (modified SBL, elastic nonwoven). Attachment strength for the 2^{nd} attachment was greater than the first attachment strength for both nonwoven fabrics. The 3^{rd}, 4^{th} and 5^{th} attachments resulted in a slight decline in peak load values.

### Evaluation of Dry versus Moist Modified Foam

As shown in the previous exampled, using a surface modified foam layer results in a doubling of the strength of the foam attachment to a number of nonwoven fabrics. While not to be bound by theory, it is believed that two potential mechanisms for this improvement may exist. First, the surface modifier may stiffen the surface of the foam and free-standing struts, and potentially increase the coefficient of friction of the surface of the foam, and therefore increase the shear strength of the foam layer/nonwoven fabric bond. The second potential mechanism may be that the surface modifier may act similar to a pressure-sensitive adhesive, providing a direct adhesive bond to the fibers of the nonwoven fabric.

Even though the surface of the coated foam was not tacky, an experiment was conducted to evaluate the mechanism of shear strength improvement. The experiment consisted of slightly moistening the surface of the coated foam layer and then measuring the attachment strength of the moist coated foam layer. The attachment strength of the moist coated foam layer was compared to the attachment strength of dry coated foam layer. Moisture in this experiment is though to act as an inhibitor of adhesive interactions, so that if the adhesive mechanism was the cause of the attachment strength increase, the increase should have been reversed and reverted to the value seen in the uncoated foam.

### Moist Shear / Peel Strength Test Method

In the moist versus dry experiment, samples of the Z65CLY foam coated with 10 gsm add-of of H9076 (Sample 2C) were submerged in water, the excess water was removed by blotting with paper towels until the samples were slightly moist. Curved Shear strength testing was conducted utilizing the test method as described above with two nonwoven fabrics (SBL, Modified SBL). Results are shown in Table 5.

**Table 5 Curved Shear Attachment Strength - Moist versus Dry**

| Sample | Peak Load, gf 1 gram-force = 0.0098N | | | n* | Energy to peak, g*cm | | |
|---|---|---|---|---|---|---|---|
| | Avg. | S. Dev. | %COV | | Avg. | S.Dev. | %COV |
| 2A - SBL - Dry | 310 | 40 | 13 | 5 | | | |
| 2C - SBL - Dry | 1677 | 124 | 7 | 4 | 11797 | 2664 | 23 |
| 2C - SBL - Moist | 1687 | 186 | 11 | 4 | 10584 | 3043 | 29 |
| 2A - Modified SBL - Dry | 1233 | 185 | 14 | 4 | | | |
| 2C - Modified SBL - Dry | 2531 | 559 | 22 | 4 | 32737 | 11790 | 36 |
| 2C - Modified SBL - Moist | 2266 | 261 | 12 | 5 | 24335 | 3081 | 13 |

| | | | | | | | |
|---|---|---|---|---|---|---|---|
| n* - number of specimen tested per sample | | | | | | | |

In addition peel testing was performed on the materials as well. The results are shown in Table 6 with the test method following.

**Table 6 Peel Attachment Strength - Moist versus Dry**

| Sample | Peak Load, gf 1 gram-force = 0.0098N | | | n* | Average Load, gf | | |
|---|---|---|---|---|---|---|---|
| | Avg. | S. Dev. | %COV | | Avg. | S.Dev. | %COV |
| 2A - SBL - Dry | 0 | | | | | | |
| 2C - SBL - Dry | 53 | 12 | 22 | 3 | 43 | 18 | 42 |
| 2C - SBL - Moist | 66 | 12 | 18 | 4 | 19 | 7 | 34 |
| 2A - Modified SBL - Dry | 0 | | | | | | |
| 2C - Modified SBL - Dry | 92 | 11 | 12 | 4 | 45 | 3 | 7 |
| 2C - Modified SBL - Moist | 71 | 19 | 27 | 2 | 38 | 16 | 43 |

| | | | | | | | |
|---|---|---|---|---|---|---|---|
| n* - number of specimen tested per sample | | | | | | | |

The shear testing results show a slight directional decrease for the moist foam layer over the dry foam layer for Modified SBL. On the other hand, the moist foam layer showed a slight directional increase for the moist foam layer over the dry foam layer for SBL.

### Peel Strength Test Method

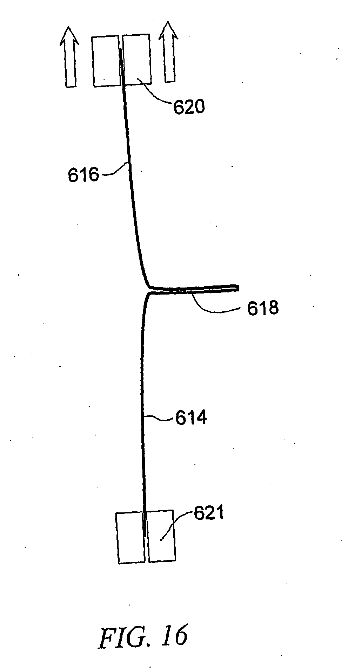

Peel tests were conducted with the universal test machine (not shown) using the 180° peel configuration shown in FIG. 16, where the foam layer and nonwoven fabric 614 and 616, respectively, are joined in an attachment zone 618 configured to be peeled apart as the remote ends of the strips 614 and 616, respectively, are moved away from each other as they are held in the jaws of an upper clamp 620 and a lower clamp 621 as shown. Using the universal testing machine (not shown) as described in the curved shear attachment test method, the force required to peel apart the attached foam layer and nonwoven fabric 614 and 616, respectively, may be measured. The crosshead speed for the peel testing was 50.8 cm (20 inches) per minute. The attachment zone 618 had a length (overlap distance) of 5.08 cm (two inches), and a width of 7.62 cm (3 inches) (38.7 cm² (6 square inches) total overlap area 612). The gauge length (distance between the upper and lower clamps 620 and 621, respectively) for the test set up was 3.81 cm (1.5 inches). The Testworks software used could not generate statistical results for peel values less than 0.098N (10 grams of force). In all cases peel values for uncoated foam was 0.

The peel testing results show that the average peel loads had a directional tendency to be lower in case of moist foam samples. The difference between moist and dry samples was not statistically significant. In case of peak peel loads, a mixed tendency was observed, peel decreased for moist foams on Modified SBL, while increasing on SBL.

The results of the shear testing and peel testing indicate no statistical difference in attachment due to moisture. Hence, it is believed the improvement in attachment of the surface modified foam layer to nonwoven fabrics is due primarily to mechanical interlocking and stiffening of the foam surface. Adhesive interactions may play a secondary, albeit less significant, role in the attachment mechanism.

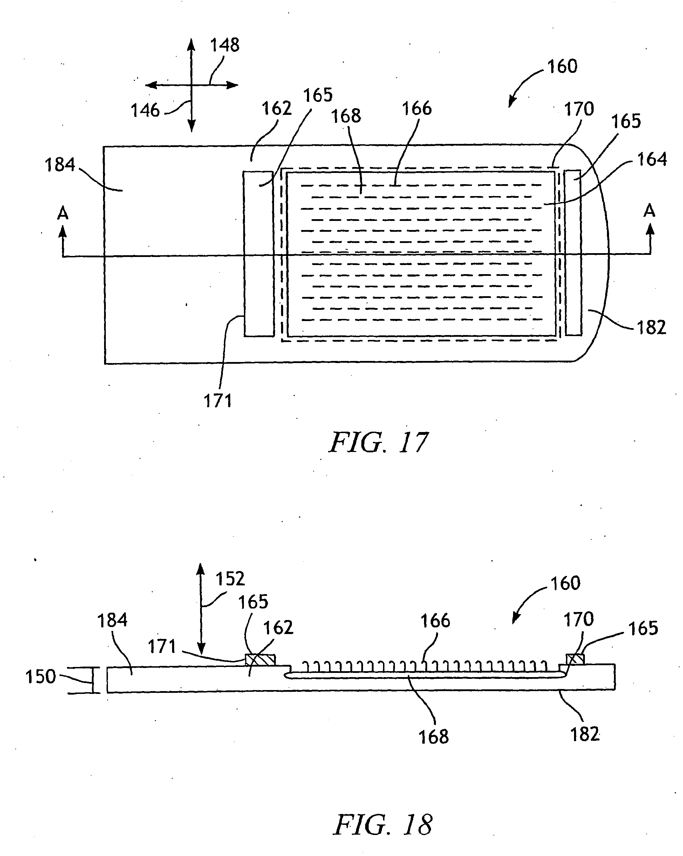

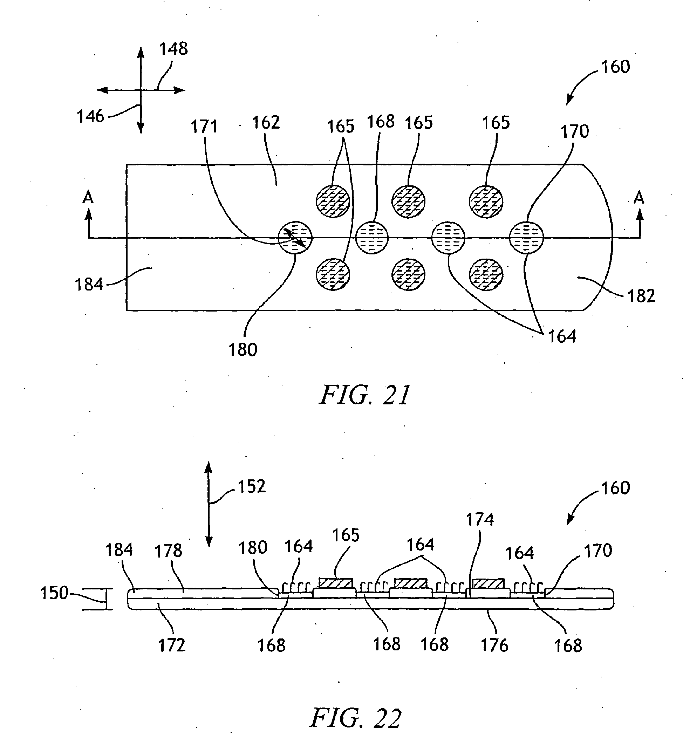

The foam layer 91 as described herein may be utilized in fastening systems including other mechanical fasteners. Figures 17, 18, 19 and 20 illustrate fasteners which are not in accordance with the invention, but have been retained to facilitate understanding of certain aspect of the invention. Figures 21, 22, 25 and 26 illustrate mechanical fasteners in accordance with the invention. For example, FIG.17 representatively illustrates an example of a cloth-like mechanical fastener not in accordance with the invention, as generally indicated at 160. As illustrated in FIG. 17, the fastener 160 comprises a flexible layer 162, a first fastener island 164 and second fastener islands 165. The flexible layer 162 may comprise an of the foam layers as described herein. The first fastener island 164 has a planar perimeter edge 170, a mechanical fastening material 166 and a backing material 168 attached to the mechanical fastening material 166. The second fastener island 165 has a planar perimeter edge 171 and a foam layer 165. The fastener 160 may also define a user's end 182, a manufacturer's bond end 184, a fastener longitudinal direction 146, and a fastener lateral direction 148. As used herein, the term "fastener longitudinal direction" means the direction that is parallel to the centerline of an absorbent article when a fastener 160 is attached to an absorbent article and generally corresponds to the "y" direction of the fastener 160. As used herein, the term "fastener lateral direction" means the direction that is perpendicular to the centerline of an absorbent article when a fastener 160 is attached to an absorbent article and generally corresponds to the "x" direction of the fastener 160. As used herein, the term "third direction" means the direction that is perpendicular to the plane defined by the fastener lateral direction and the fastener longitudinal direction, and generally corresponds to the "z" direction of the fastener 160. As used herein, the term "planar perimeter edge" means the outermost edge of the first fastener island 164 along a plane defined by the lateral 148 and longitudinal direction 146, and is perpendicular to the third direction 152. As such, the planar perimeter edge 170 defines the edge of the first fastener island 164 at its largest cross section.

The illustrated mechanical fastener 160 includes a flexible layer 162. The flexible layer 162 generally provides the chassis for the fastener 160. The flexible layer 162 desirably provides a feeling of flexibility and softness to the wearer. When the flexible layer comprises a foam layer as described herein, the flexible layer may also contribute to the fastening characteristics of the mechanical fastener 160. In some embodiments, the flexible layer 162 may be provided by a variety of materials as are well known to those skilled in the art. For example, the flexible layer 162 may be provided by knits, wovens, fabrics, papers, nonwovens, and similar materials, or combinations thereof. Various types of nonwoven materials may be advantageously used as the flexible layer 162, such as a thermally or chemically bonded carded web or a nonwoven laminate. Examples of nonwoven laminates that may be advantageously used as the flexible layer 162 include stretchable neck bonded laminates, such as those disclosed in U.S. Patent No. 5,789,065 issued on August 4, 1998 to Haffner et al. and U.S. Patent No. 5,336,545 issued on August 9, 1994 to Morman. Alternatively, relatively inelastic nonwoven laminates, such as a spunbond/meltblown/spunbond composite may also be advantageously used. The flexible layer 162 may be provided by a nonwoven such as a neck bonded laminate or a thermally bonded carded web (hereinafter "TBCW"). In particular, it is desirable that the fibers of the flexible layer 162 be sufficiently fine such that the flexible layer 162 is accordingly soft to the touch.

As representatively illustrated in FIGS. 18, 20 and 22, the fastener 160 also defines a fastener thickness 150 in a third direction 152. The flexible layer 162 of the fastener 160 may define a fastener thickness 150 which is generally smaller than the thickness of the chassis of fasteners known in the art, alternatively, the flexible layer 162 of the fastener 160 may define a fastener thickness 150 which is generally larger than the thickness of the chassis of fasteners known in the art. Desirably, however, the total fastener thickness 150 of the flexible layer 162 remains generally greater than the thickness of the first fastener island 164 in the third direction 152. In particular, the flexible layer 162 may define a fastener thickness 150 of from about 250 µm to about 5000 µm. More particularly, the flexible layer 162 may define a fastener thickness 150 of from about 1000 µm to about 4000 µm. Yet even more particularly, the flexible layer 162 may define a fastener thickness 150 of from about 2000 µm to about 3500 µm. In particular aspects, the fastener thickness 150 provided by the flexible layer 162 can be at least a minimum of about 250 µm. The fastener thickness 150 can alternatively be at least about 400 µm, and optionally, can be at least about 600 µm to provide improved performance. In other aspects the fastener thickness 150 provided by the flexible layer 162 can be not more than a maximum of about 3500 µm. The fastener thickness 150 can alternatively be not more than about 1600 µm, and optionally, can be not more than about 1200 µm to provide improved performance. As such, the flexible layer 162 maintains in the fastener 160 a desirable flexibility and drape to provide the wearer and the caregiver with the sensation of softness and comfort, such as would be expected to be provided by a cloth-like material.

The flexible layer 162 of the fastener 160 generally provides the shape of the fastener 160. That is, the perimeter edge of the flexible layer 162 defines the profile or shape of the fastener 160. As such, the fastener 160 may have a variety of suitable shapes as are well known to those in the art. For example, as representatively illustrated in FIGS. 17-22 and 24, the fastener 160 may have a generally rectangular shape. Alternatively, the flexible layer 162 may provide the fastener 160 with a curvilinear shape that may improve the comfort of the wearer by better conforming to the contours of the wearer's body.

Desirably, the flexible layer 162 is extensible or elastic in at least the fastener lateral direction 148. For example, the flexible layer 162 may be comprised of a stretch-thermal laminate (STL) neck-bonded laminate (NBL), or stretch-bonded laminate (SBL) material. Methods of making such materials are well known to those skilled in the art and described in U.S. Patent No. 4,663,220 issued May 5, 1987 to Wisneski et al., U.S. Patent No. 5,226,992 issued July 13, 1993 to Morman, and European Patent Application No. EP 0 217 032 published on April 8, 1987 in the names of Taylor et al.

The flexible layer 162 may include a single piece of material or multiple pieces of material. For example, the flexible layer 162 may include multiple pieces of material in the fastener lateral direction 148. As such, the flexible layer 162 may include an extensible panel located between a pair of generally non-extensible flexible materials to provide a flexible layer 162 that is extensible, as described above. Alternatively, the flexible layer 162 may include multiple pieces of material that are arranged in layers in the third direction 152, as will be discussed in more detail below.

The mechanical fastener 160 further includes at least one discrete first fastener island 164. In accordance with the invention, the mechanical fastener includes a plurality of discrete first fastener islands. As representatively illustrated in FIGS. 17-21 the discrete first fastener island 164 includes a mechanical fastening material 166 and a backing material 168 attached to the fastening material 166. The first fastener island 164 also defines a planar perimeter edge 170. The planar perimeter edge 170 is the outermost edge of the first fastener island 164 along a plane that is perpendicular to the third direction 152. As such, the planar perimeter edge 170 defines the edge of the first fastener island 164 at its largest cross section.

The mechanical fastener 160 may include at least one discrete second fastener island 165. In accordance with the invention, the mechanical fastener includes a plurality of discrete second fastener islands. As representatively Illustrated in FIGS. 17-21 the discrete second fastener island 165 includes a foam fastening material as described herein. The second fastener island 165 also defines a planar perimeter edge 171. The planar perimeter edge 171 is the outermost edge of the second fastener island 165 along a plane that is perpendicular to the third direction 152. As such, the planar perimeter edge 171 defines the edge of the second fastener island 165 at its largest cross section.

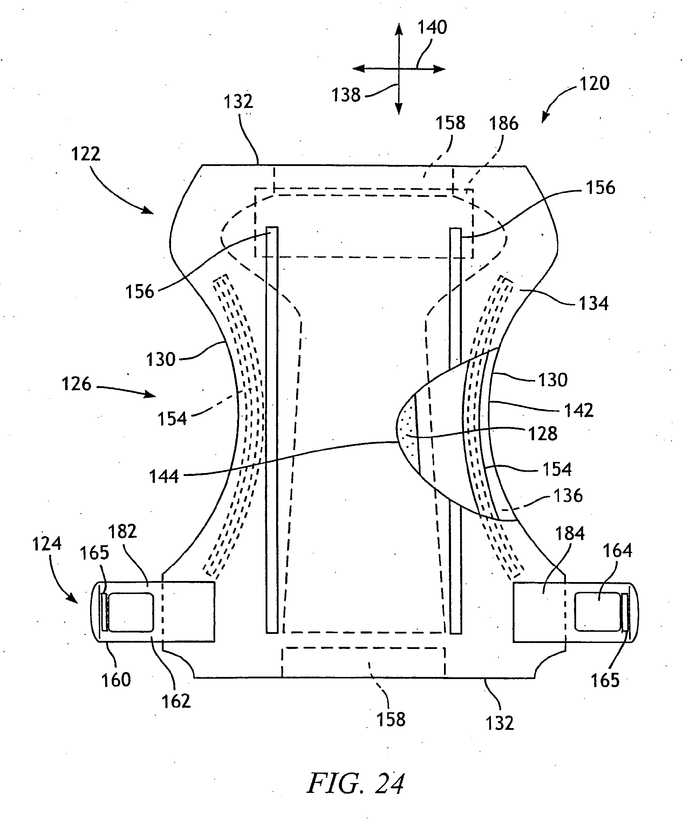

The mechanical fastening material 166 of the discrete first fastener island 164 allow the fastener 160 to refastenably engage the exterior surface 136 of the diaper 120 (shown in FIG. 24), thereby securing the diaper 120 about the wearer in use. Suitable fasteners to provide the fastening material 166 of the fastener islands 164 are well known to those skilled in the art and can include, hook and loop material, mushroom material, snaps, pins, and similar fastening material, and combinations thereof. In accordance with the invention, the fastening material 166 of the first fastener island 164 is a hook type fastener material. As such, the first fastener island 164 may contain multiple hooks. For example, as representatively illustrated in FIGS. 17-22 and 25, the fastening material 166 of each of the fastener islands 164 provides multiple hooks. In particular, the fastening material 166 of each of the fastener islands 164 may contain at least about 20 hooks.

The foam fastening material of the second discrete fastener island 165 assists the mechanical fastening material 166 in securing the diaper 120 about the wearer in use. However, because foam material is generally softer, less stiff and more skin friendly than mechanical fastening, this added fastening may be provided closer to the edge of the fastener 160, in locations that may come in contact with the skin.

The number of hooks can also be described in terms of a hook density (number of hooks per square centimeter). It is possible to fabricate hook material having a hook density of from about 60 hooks/cm² to about 1600 hooks/cm². More desirably, the hook material has a hook density of from about 100 hooks/cm² to about 750 hooks/cm². The term "hook" should be understood to encompass various geometries of protuberances that are suitable for engaging into a loop material or a material having loop characteristics in order to place or secure a fastener. Exemplary geometries include prongs, stems, trees (such as the shapes connoted by "evergreen" and "palm" trees), mushrooms, J-hooks, bidirectional hooks and studs protruding at various angles. In addition to the various possible geometries of hooks, the hooks may protrude from a backing material at various angles. U.S. Patent No. 5,782,819 issued to Tanzer et al. on July 21, 1998 describes a fastener system that includes velvet fabrics as examples of materials exhibiting differential friction. The surface of velvet fabric has fibers protruding from the surface, oriented on a bias. Despite the fibers being essentially straight (i.e. without barbs or hooks), they engage an opposed surface and facilitate fastening. The discrete hooks of the hook material may include or be treated with materials such as soft rubbers that increase the coefficient of friction of the hooks against the corresponding loop/engaging material. The increased coefficient of friction serves to reduce the tendency of the fastener to pop-open under stress. The benefits of fasteners having increased coefficients of friction are described in U.S. Patent Application Serial No. 09/705,512 entitled "Hook and Loop Fastener Having an Increased Coefficient of Friction" filed by Martin et al. on November 3, 2000.

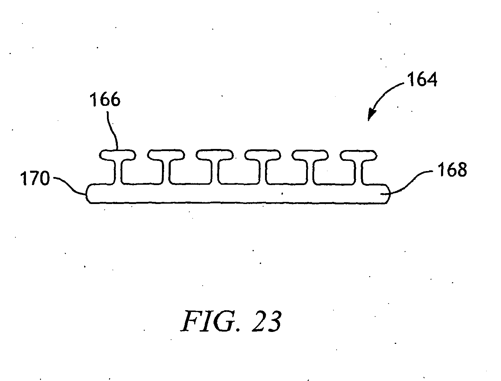

In embodiments of the invention in which the mechanical fastening material 166 of the first fastener island 164 is provided by hook material, different hook configurations may be provided. For example, as representatively illustrated in FIG. 23, the fastening material 166 may be provided by a flat top hook material. Flat top hook material advantageously presents a surface that is less likely to expose the wearer to any coarse, sharp edges and provides a more smooth feeling fastener surface. As such, the flat top hook material provides a fastening material 166 that may reduce the possibility of irritation and discomfort to the wearer and/or the caregiver. In addition, the flat top hook material advantageously provides reliable engagement with the exterior surface 136 of the diaper 120, ensuring that the mechanical fasteners 160 will dependably refastenably secure the diaper 120 about the waist of a wearer, as will be described in greater detail below.

The first fastener islands 164 also include a backing material 168 that is attached to the fastening material 166. Alternatively the backing material 168 of the first fastener islands 164 may be embedded within the flexible layer 162 of the fastener. By embedding the backing material 168 of the fastener islands 164 within the flexible layer 162, the present invention provides the wearer with a more cloth-like fastener in that there is a reduced possibility of irritation and discomfort because the rigid edges of the first fastener island 164 are recessed within the flexible layer 162. As such, the embedding of the backing material 168 of the fastener islands 164 also ensures that the planar perimeter edge 170 of the first fastener Island 164 is surrounded by the flexible layer 162. Accordingly, the only portion of the first fastener island 164 that is exposed above the surface of the flexible layer (in the "z" direction) is the fastener material 166. This configuration ensures that the fastener 160 is able to provide a cloth-like presentation and reduces the possibility of irritation and discomfort to the wearer.

The fastener islands 164 may be embedded within the flexible layer 162 in a variety of ways. For example, as representatively illustrated in FIGS. 17-18, the first fastener island 164 may be provided by applying molten polymer to the flexible layer 162. The drops of molten polymer may then be molded into a discrete first fastener island 164. As such, during the molding process, some of the polymer may impregnate a discrete section of the nonwoven web forming the backing material 168 of the first fastener island 164, while some other portion of the polymer is molded into the mechanical fastening material 166 of the first fastener island 164. For example, the mechanical fastening material 166 may be molded into hooks. The molten polymer may then be chilled, providing a flexible layer 162 with the backing material 168 of the first fastener island 164 embedded therein.

Alternatively, as representatively illustrated in FIGS. 21 and 22, the embedding of the first fastener island 164 within the flexible layer 162 may be accomplished by providing the flexible layer 162 with multiple layers in the third direction 152. For example the flexible layer 162 may be comprised of a first flexible layer 172 and a second flexible layer 178. The first flexible layer 172 defines an interior surface 174 and an exterior surface 176 opposite the interior surface 174. The second flexible layer 178 can be attached to the interior surface 174 of the first flexible layer 172. Similarly the backing material 168 of the first fastener island 164 is permanently attached to the first flexible layer interior surface 174. The second flexible layer 178 defines an opening 180 which corresponds to each of the fastener islands 164. The opening 180 in the second flexible layer 178 allows the mechanical fastening material 166 of the first fastener island 164 to be exposed while the backing material 168 remains embedded within the second flexible layer 178.

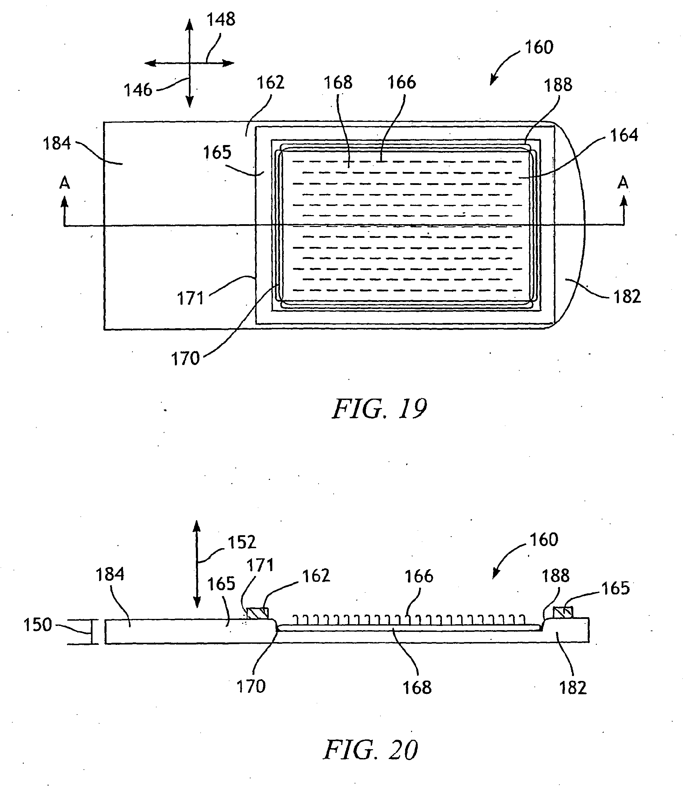

In yet another alternative, the fastener islands 164 of the present invention may be embedded within the flexible layer 162 of the fastener 160 by ultrasonic bonds. For example, as representatively illustrated in FIGS. 19 and 20, the first fastener island 164 is permanently attached to the flexible layer 162 using ultrasonic bonds 188. In particular, by using closely spaced ultrasonic bonds 188, the backing material 168 of the first fastener island 164 becomes recessed within the flexible layer 162. For example, each first fastener island 164 can have one or more bond points for holding it in place. Accordingly the fastener 160 may thereby provide a more cloth-like presentation that has a reduced possibility of irritating the wearer's skin.

In another aspect, the present invention includes fasteners 160 in which the flexible layer 162 is a soft, flexible foam with a density of less than about 0.4 g/cm³. The fastener islands 164 are applied to the top surface of the flexible layer 162. The fastener islands 164 are sonically bonded to the flexible layer 162. During the process of sonic bonding, the foam of the flexible layer 162 is partially crushed, thereby reducing its thickness approximately in half and approximately doubling its density. Alternatively, the flexible layer 162 can include three or more layers. With the multiple-layered flexible layer 162 of the invention, there is a first flexible layer 172 having an interior surface 174 and an exterior surface 176. An adhesive is applied to the interior surface 174 of the first flexible layer 172. The backing material 168 of the fastener islands 164 is applied to the adhesive-coated interior surface 174. The backing material 168 can include flanges that extend laterally away from the positions of the individual hooks. Such flanges can serve to further anchor the backing material 168 to the first flexible layer 172. The flexible layer 162 further includes a second flexible layer 178 that has pre-cut holes or openings 180 that correspond to the locations of the fastener islands 164. The second flexible layer 178 is applied onto the first flexible layer 172 over the fastener islands 164. It is also possible for the flanges to extend between fastener islands 164 so that the fastener islands 164 are the intersections. The second flexible layer 172 may comprise any of the foam layers as describe herein. In such an aspect, the first flexible layer 172 can be substantially thinner than the second flexible layer 178. For example, the first flexible layer 172 can include a spunbond layer having a basis weight of about 20 to about 40 g/m².

In embodiments of the invention, the fastener 160 includes second discrete fastener islands 165. The second discrete fastener islands 165 may have a planar perimeter edge 171 and a foam fastening layer as disclosed herein. Where the flexible layer 184 includes a first flexible layer 172 and a second flexible layer 178, the second discrete fastener islands 165 may be attached to the first flexible layer 172 with corresponding holes is the second flexible layer 178, alternatively, the second discrete fastener islands 165 may be attached to the second flexible layer 178.

The present invention also encompasses different heights above the flexible layer 162 that the mechanical fastening material 166 is exposed. One method of varying the height that the mechanical fastening material 166 is exposed in to vary the softness of the flexible layer 162. If the compression modulus of the flexible layer 162 is low (relative to how much force is used when the fastener 160 is applied during use), it is possible for the top of the mechanical fastening material 166 to be even with the "top" surface of the flexible layer 162. The greater the compression modulus of the flexible layer 162, the more of the mechanical fastening material 166 that must be exposed for adequate hook engagement. One advantage of having the top surface of the mechanical fastening material 166 even with the flexible layer 162 is that the fastener 160 would have a very gentle feel and any non-engaged portion of the mechanical fastening material that contacts skin would not have exposed hook members.

A second method for varying the height that the mechanical fastening material is exposed 166 is by varying the thickness of the second discrete fastener islands 165. By having a relatively thin second discrete fastener islands 165, a relatively large amount of the mechanical fastening material 166 will be exposed. Correspondingly, by having a relatively thick second discrete fastener islands 165, a relatively small amount of the mechanical fastening material 166 will be exposed, or the mechanical fastening material 166 will be below the level of the top of the second discrete fastener islands 165.

The mechanical first fastener island 164 may be provided in a variety of suitable shapes as are well-known to those skilled in the art. For example, as representatively illustrated in FIGS. 19 and 20, the first fastener island 164 has a generally rectangular shape. Alternatively, as representatively illustrated in FIGS. 21 and 22, the first fastener island 164 presents a generally circular shape. Other suitable shapes may includes, but are not limited to, triangular, oval, linear, and the like, or combinations thereof. It is desirable to use a shape of mechanical first fastener island 164 that does not have sharp edges and, if the mechanical fastener islands 164 are formed from a strip of material, to use a shape that "nests" so as to minimize material waste.

The second discrete fastener island 165 may be provided in a variety of suitable shapes as are well-known to those skilled in the art. For example, the second discrete fastener island 165 may have shapes similar to the first discrete fastener islands 164. Suitable shapes may include, but are not limited to, triangular, oval, linear, and the like, or combinations thereof. It is desirable to use a shape of the second fastener island 165 that does not have sharp edges and, if the second fastener islands 165 are formed from a strip of material, to use a shape that "nests" so as to minimize material waste.

As described above, the mechanical fastener 160 may be provided with at least one first fastener island 164 attached to the flexible layer 162 and one second fastener island 165 attached to the flexible layer 162. As representatively illustrated in FIG. 21 and 22 in accordance with the invention the fastener 160 includes a plurality of fastener islands 164, 165. For example, as representatively illustrated in FIGS. 21 and 22 the mechanical fastener 160 includes multiple fastener islands 164. As such, the mechanical fastener 160 is provided with even greater flexibility. This increased flexibility is provided by having some flexible layer 162 material located between the multiple fastener islands 164. Therefore, a fastener with multiple fastener islands 164 is more flexible than a fastener that must be bent without multiple fastener islands 164. The backing material 168 is typically substantially stiffer than the nonwoven material typically used for the flexible layer 162. By breaking the mechanical Fastener material 166 into discrete islands, the nonwoven material of the flexible layer 162 acts as a hinge. Moreover, since the multiple fastener islands 164 reduce the possibility of the user of the fastener 160 from creasing the backing material 168 of the fastener islands 164, the opportunity for the creation of harsh edges in the fastener 160 is reduced. Finally, the reduction of the possibility for harsh edges, which may develop in a traditional mechanical fastener in use, likewise reduces the opportunity for the fastener to red-mark or irritate the wearer's skin. In addition, the plurality of second fastener islands 165 which comprise foam material may secure the edges of the fastener 160 with a reduced risk of the mechanical fastening material 166 coming in contact with a user's skin.

The increased flexibility of the mechanical fastener 160 with multiple fastener islands 164 also allows the mechanical fastener 160 to be adjusted to a wider range of positions in use to achieve the optimum fastening location on the diaper 120 for improved fit and comfort. For example, a more flexible fastener may be capable of engaging the exterior surface 136 of the diaper 120 in a wider range of locations than a more rigid fastener. That is, the fastener 120 of the present invention is capable of being extended and bent more easily than a rigid mechanical fastener. A rigid mechanical fastener may have a more limited range of motion and thus a more limited area of engagement locations on the diaper 120. As such, a more flexible fastener such as the fasteners 160 of the present invention may be used to improve the fit and comfort of the wearer of the diaper 120 in use and thereby also reduce the opportunity for undesirable leakage. Moreover this added flexibility allows the fastener 160 to better accommodate the movement of the wearer in use.

In a particular embodiment, as representatively illustrated in FIGS. 21 and 22, the mechanical fastener 160 may include a plurality of generally circular first and second discrete fastener islands 164, 165. As such, the discrete fastener islands 164, 165 may define a fastener island diameter 171. Desirably, the fastener island diameter 171 is from about 8 mm to about 32 mm. Even more desirably, the fastener island diameter 171 is from about 10 mm to about 28 mm, and still yet more desirably, the fastener island diameter 171 is from about 14 mm to about 20 mm. In particular aspects, the fastener island diameter 171 can be at least a minimum of about 8 mm. The fastener island diameter 171 can alternatively be at least about 10 mm, and optionally, can be at least about 14 mm to provide improved performance. In other aspects, the fastener island diameter 171 can be not more than a maximum of about 28 mm, and optionally, can be not more than about 20 mm to provide improved performance.

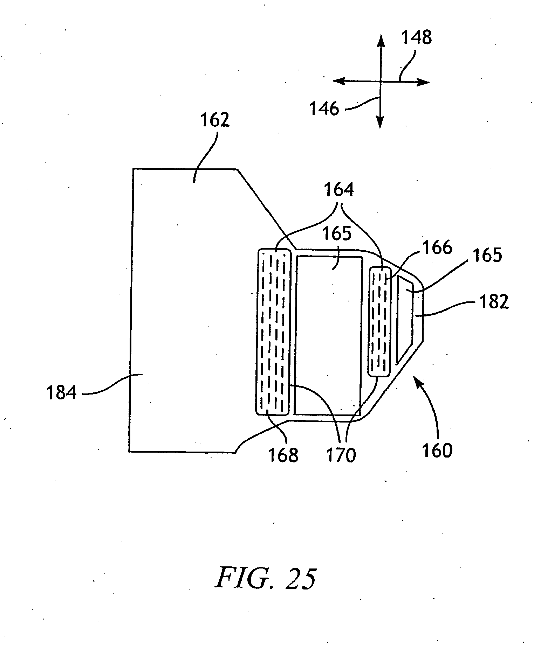

In a particular aspect, as representatively illustrated in FIG. 25, the mechanical fastener 160 of the present invention may include a plurality of first and second discrete fastener islands 164, 165 where the flexible layer 162 is extensible between each of the fastener islands 164, 165. Even more particularly, there may be a pair of first fastener islands 164 that extend substantially along the entire fastener 160 in the fastener longitudinal direction 146, while yet being relatively narrow in the fastener lateral direction 148, and a pair of second fastener islands 165 that extend substantially along the entire fastener 160 in the fastener longitudinal direction 146, while yet being relatively narrow in the fastener lateral direction 148. The first and second fastener island 164, 165 alternating in the lateral direction. Accordingly, this particular embodiment may be directed to a mechanical fastener 160 having a fastener islands 164, 165 that extend generally in the fastener longitudinal direction 146 and not as extensively in the fastener lateral direction 148, and having a flexible layer 162 which is extensible particularly between the fastener islands 164, 165. This arrangement, when applied in a stretched configuration, acts to pull the fastener islands 164, 165 together, thereby placing the mechanical fastener 160 in a shear mode of failure in use. As such, this particular embodiment advantageously provides a mechanical fastener 160 that is subjected primarily to shear forces when engaged upon the exterior surface 136 of a diaper 120. Typically, a fastener that is subjected primarily to shear forces provides more reliable securement than a fastener that is subjected primarily to peel forces in use. As such, the mechanical fastener 160 of this particular embodiment is capable of providing increased securement with a smaller amount of fastener material 166, thereby providing improved performance at a reduced material cost.

Still more particularly, the fastener islands 164, 165 of this specific aspect of the mechanical fastener 160 described above may have a particular length in the fastener lateral direction 148. For example, the length of the fastener islands 164, 165 in the fastener lateral direction 148 may desirably be from about 0.625 cm to about 2.54 cm. Even more desirably, the fastener islands 164, 165 may have a length in the fastener lateral direction 148 of about .95 cm. In particular aspects, the length of the fastener island 164, 165 in the fastener lateral direction 148 can at least be a minimum of about .625 cm. In other aspects, the length of the fastener island 164, 165 in the fastener lateral direction 148 can be not more than a maximum of about 2.54 cm to provide improved performance.

The number and configuration of fastener islands 164, 165 on the fasteners 160 of the invention can vary. A moderate number of fastener islands 164, 165 on a fastener 160 can range from to 2 to about 16; a large number of fastener islands 164, 165 on a fastener 160 would be a number greater than about 16. In addition to the number of fastener islands 164, 165, the total area accumulated by the fastener islands 164, 165 will affect the cost, flexibility, grip, skin friendliness and ease of manufacture of the fasteners 160. A low area is an area of about 2 cm² or less; a high area is an area of about 8 cm² or more; a moderate area is an area between about 2 cm² and about 8 cm². Having a relatively low number of islands 164, 165 combined with a low area provides a fastener 160 having low manufacturing cost, high flexibility, low grip and skin friendliness. Increasing the area to a moderate hook area increases the cost and improves the grip of the fastener 160; using a high area with a low number of islands 164, 165 would have a further increased cost. Having a relatively large number of islands 164, 165 combined with a low area provides a fastener 160 having low manufacturing cost, high flexibility, low grip and skin friendliness but also being relatively more difficult to manufacture at high speeds. Increasing the hook area to a moderate area increases the cost and improves the grip of the fastener 160; using a high area with a large number of islands 164 would have an even higher cost and could have decreased skin friendliness. Based on a balancing of the relevant factors, it is desirable for a fastener 160 to have a relatively low number of fastener islands 164, 165 and a moderate total area (the area of first and second fastener islands not including the "sea" areas between the fastener islands 164, 165). Such fasteners 160 provide the benefits of moderate cost, high flexibility, strong grip and skin friendliness.

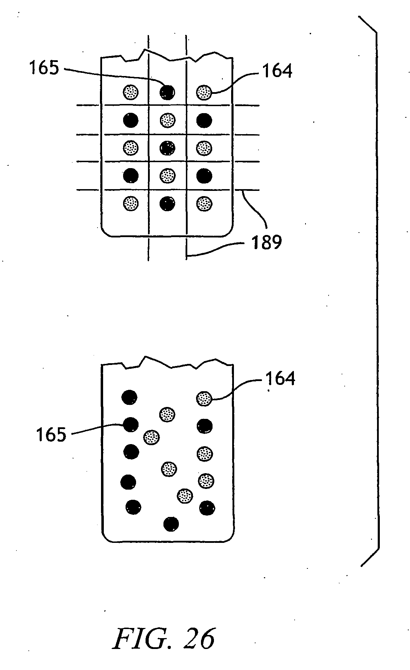

The spacing between fastener islands 164, 165 can range from about 3 mm to about 30 mm. The fastener islands 164, 165 can be arranged in any suitable geometry including a "checkerboard" pattern, a chevron pattern and around the perimeter of an oval or other shape. For some fasteners 160, it may be desirable to arrange the fastener islands 164, 165 to create well-defined lines of flexibility by leaving "lines" free of fastener islands 164, 165. For other fasteners 160, it may be desirable to arrange the fastener islands 164, 165 to block lines of flexibility. FIG. 26 depicts two embodiments of fasteners of the present invention: one embodiment shows the fastener islands 164, 165 arranged to create well-defined lines of flexibility 189 while the other embodiment shows the fastener islands 164 arranged so as to block lines of flexibility 189.

The mechanical fastening material 166 of the discrete first fastener islands 164 of this embodiment of the present invention are a hook fastener material, as already described in detail herein. In particular, the fastening material 166 may be VELCRO HTH 858 or VELCRO HTH 823, or a similar hook material.

The various components of the fastener 160 are integrally assembled together employing various types of suitable attachment means known in the art, such as adhesive, sonic and thermal bonds or combinations thereof. It is generally desirable to have the majority of the components of the fastener 160 be assembled together using ultrasonic bonding techniques for reduced manufacturing cost. For example, as discussed in more detail herein, the planar perimeter edge 170 of the first fastener island 164 may be embedded within the flexible layer 162 of the fastener 160 by various attachment means, including sonic bonding.

As representatively illustrated in FIGS. 17-22 and 25, the flexible cloth-like mechanical fastener 160 of the present invention may further define a manufacturer's bond end 184 and a user's end 182. As used herein, reference to a manufacturer's bond end 184 is intended to refer to that portion of a fastener which is attached to the diaper 120 by the manufacturer of the diaper as part of the diaper production process. That is, the manufacturer's bond end 184 is generally intended to be permanently attached to the diaper 120. Likewise, as used herein, reference to a user's end 182 is intended to refer to that portion of the fastener 160 that is used by the wearer or caregiver to secure the diaper 120 about the waist of the wearer, and which generally includes the discrete fastener islands 164, 165. The user's end 182 of the mechanical fastener 160 is generally designed to be refastenable such that the diaper can be fastened and refastened about a wearer through the use of the user's end 182 of the mechanical fastener 160. Thus, the attachment formed by the user's end 182 of the mechanical fastener 160 is generally nonpermanent.

Methods of bonding the fastener 160 to the diaper 120 to define the bond end 184 are well known to those skilled in the art. For example, as representatively illustrated in FIG. 24, the mechanical fasteners 160 may be permanently adhered to the side edges 130 of the diaper 120 by adhesive bonds, sonic bonds, thermal bonds, and the like, or combinations thereof. As discussed above, the method of attachment used to form the bond end 184 is generally intended to be permanent. Desirably, the bond end 184 is attached to the diaper 120 using ultrasonic bonding techniques for reduced manufacturing cost.

FIG. 24 representatively illustrates the mechanical fastener 160 included in combination with a disposable diaper 120. In particular, the diaper 120 is shown in an unfastened, stretched and laid flat configuration with the surface of the diaper adapted to contact the wearer's skin facing the viewer and with portions of the diaper partially cut away to show the underlying features. The illustrated diaper 120 defines an absorbent core 128, a front waist region 122, a back waist region 124, a crotch region 126 which extends between and connects the front and back waist regions 122 and 124, a longitudinal direction 138 and a lateral direction 140. As used herein, the term "longitudinal direction" means the direction that is parallel to the machine direction of the diaper 120 and generally corresponds to the "y" direction of the diaper 120. As used herein the term "lateral direction" means the direction that is perpendicular to the machine direction of the diaper 120 and generally corresponds to the "x" direction of the diaper 120. The front waist region 122 includes the portion of the diaper 120 which, when worn, is positioned on the front of the wearer while the back waist region 124 comprises the portion of the diaper 120 which, when worn, is positioned on the back of the wearer. The crotch region 126 of the diaper 120 includes the portion of the diaper 120 which, when worn, is positioned between the legs of the wearer and covers the lower torso of the wearer.

The diaper 120 defines a pair of laterally opposed side edges 130, a pair of longitudinally opposed waist edges 132, an interior surface 134 which is configured to contact the wearer, and an exterior surface 136 opposite the interior surface 134 which is configured to contact the wearer's clothing in use. The illustrated diaper 120 also includes an outer cover 142 and a bodyside liner 144 which is connected to the outer cover 142 in a superposed relation. An absorbent core 128 is located between the outer cover 142 and the bodyside liner 144. The laterally opposed side edges 130 of the diaper 120 are generally defined by the side edges of the outer cover 142 which further define leg openings which may be curvilinear. The waist edges 132 of the diaper 120 are generally defined by the waist edges of the outer cover 142 and define a waist opening which is configured to encircle the waist of the wearer when worn. The absorbent core 128 is configured to contain and/or absorb any body exudates discharged from the wearer. The diaper 120 may further include leg elastics 154, containment flaps 156 and waist elastics 158 as are known to those skilled in the art. It should be recognized that individual components of the diaper 120 may be optional depending upon the intended use of the diaper 120.

Desirably, the fasteners 160 of the present invention may be refastenably engaged directly with the exterior surface 136 of the diaper 120 to refastenably apply the diaper about the lower torso of the wearer. Alternatively, the diaper 120 may further include an attachment panel 186. The attachment panel 186 may be located on the front or back waist region 122 and 124 respectively, opposite the waist region 122 or 124 to which the fasteners 160 are attached. As such, the attachment panel 186 may provide an alternative surface to which the mechanical fasteners 160 may be releasably engaged to form the refastenable diaper 120. For example, in FIG. 24, the attachment panel 186 is shown in phantom lines on the exterior surface 136 of the diaper 120 in the front waist region 122. In another aspect of the present invention, the mechanical fastener 160 is located within the attachment panel 186. The material into which the mechanical fastener 160 engages, such as a loop material, is then located on a lateral extension of the outer cover, such as the location where the fasteners are conventionally attached.

As previously described herein, particular embodiments of the fastener 160 of the present invention, when used in combination with the diaper 120, may improve the fit and comfort of the diaper 120. For example, the improved flexibility of the fasteners of the present invention may reduce the opportunity for the creation of harsh edges in the fastener 160, which may develop in a traditional mechanical fastener in use. As such, the possibility of the fastener red-marking or irritating the wearer's skin is decreased. Moreover, the increased flexibility of the mechanical fastener 160 allows the mechanical fastener 160 to be adjusted to a wider range of positions in use to achieve the optimum fastening location on the diaper 120 for improved fit and comfort.

Desirably, the mechanical fasteners 160 of the present invention are permanently attached to the back waist region 124 of the diaper 120, and refastenably engage the diaper 120 in the front waist region 122 increasing the ease with which the wearer or the caregiver can adjust the fit of the diaper 120. Alternatively, the fasteners 160 may be permanently attached to the front waist region 122 of the diaper 120 and refastenably engage the diaper in the back waist region 124. Such a configuration may be desirable for making the fasteners 160 more difficult for the wearer to access, thereby reducing the opportunity for the wearer to open and remove the diaper 120.

The diaper 120 may be of various suitable shapes. For example, in the unfastened configuration as illustrated in FIG. 24, the diaper may have an overall rectangular shape, T-shape or a generally I-shape. In the shown embodiment, the diaper 120 has an approximately hourglass shape in an unfastened configuration. Examples of diaper configurations suitable for use in connection with the instant application and other diaper components suitable for use on diapers are described in U.S. Patent 4,798,603 issued January 17, 1989, to Meyer et al.; U.S, 5,176,668 issued January 5, 1993, to Bernardin; U.S. 5,176,672 issued January 5, 1993, to Bruemmer et al.; U.S. 5,192,606 issued March 9, 1993, to Proxmire et al., and U.S. 5,509,915 issued April 23, 1996, to Hanson et al. The various aspects and configurations of the invention can provide distinctive combinations of softness, body conformity, reduced red-marking of the wearer's skin, reduced skin hydration, improved containment of body exudates and improved aesthetics.

The various components of the diaper 120 are integrally assembled together employing various types of suitable attachment means, such as adhesive, sonic and thermal bonds or combinations thereof. In the shown embodiment, for example, the outer cover 142 and bodyside liner 144 are assembled to each other and to the absorbent core 128 with adhesive, such as a hot melt, pressure-sensitive adhesive. The adhesive may be applied as a uniform continuous layer of adhesive, a patterned layer of adhesive, a sprayed pattern of adhesive, or an array of separate lines, swirls or dots of adhesive. Alternatively, the absorbent core 128 may be connected to the outer cover 142 using conventional fasteners such as buttons, hook and loop type fasteners, adhesive tape fasteners, and the like. The other components of the diaper 120 may be suitably connected together using similar means. Similarly, other diaper components, such as the elastic members 154 and 158 and the fasteners 160, may be assembled into the diaper 120 article by employing the above-identified attachment mechanisms. Desirably, the majority of the diaper components are assembled together using ultrasonic bonding techniques for reduced manufacturing cost.

The outer cover 142 of the diaper 120, as representatively illustrated in FIG. 24, may suitably be composed of a material which is either liquid permeable or liquid impermeable. It is generally preferred that the outer cover 142 be formed from a material which is substantially impermeable to liquids. A typical outer cover can be manufactured from a thin plastic film or other flexible liquid-impermeable material. For example, the outer cover 142 may be formed from a polyethylene film having a thickness of from about 0.013 millimeter (0.5 mil) to about 0.051 millimeter (2.0 mils). If it is desired to present the outer cover 142 with a more cloth-like feeling, the outer cover 142 may comprise a polyolefin film having a nonwoven web laminated to the exterior surface thereof, such as a spunbond web of polyolefin fibers. For example, a stretch-thinned polypropylene film having a thickness of about 0.015 millimeter (0.6 mil) may have thermally laminated thereto a spunbond web of polypropylene fibers. The polypropylene fibers have a thickness of about 1.5 to 2.5 denier per filament, which nonwoven web has a basis weight of about 17 grams per square meter (0.5 ounce per square yard). The outer cover 142 may otherwise include bicomponent fibers such as polyethylene/polypropylene bicomponent fibers. Methods of forming such cloth-like outer covers are known to those skilled in the art.

Further, the outer cover 142 may be formed of a woven or nonwoven fibrous web layer which has been totally or partially constructed or treated to impart a desired level of liquid impermeability to selected regions that are adjacent or proximate the absorbent core 128. Still further, the outer cover 142 may optionally be composed of a micro-porous "breathable" material which permits vapors to escape from the absorbent core 128 while still preventing liquid exudates from passing through the outer cover 142. For example, the outer cover 142 may include a vapor permeable non-woven facing layer laminated to a micro-porous film. Suitable "breathable" outer cover materials are described in U.S. Patent No. 5,695,868 issued to McCormack et al. and U.S. Patent No. 5,843,056 issued December 1, 1998 to Good et al. Still further, the outer cover 142 may also be an elastomeric material such as a stretch-thermal laminate (STL), neck-bonded laminate (NBL), or stretch-bonded laminate (SBL) material. Methods of making such materials are well known to those skilled in the art and are described in U.S. Patent No. 4,6663,220 issued May 5, 1987 to Wisneski et al., U.S. Patent No. 5,226,992 issued July 13, 1993 to Mormon, and European Patent Application No. EP 0 217 032 published on April 8, 1987 in the names of Taylor et al. The outer cover 142 can also be embossed or otherwise provided with a matte finish to provide a more aesthetically pleasing appearance.

The bodyside liner 144, as representatively illustrated in FIG. 24, suitably presents a bodyfacing surface which is compliant, soft feeling, and nonirritating to the wearer's skin. Further, the bodyside liner 144 may be less hydrophilic than the absorbent core 128, to present a relatively dry surface to the wearer, and may be sufficiently porous to be liquid permeable, permitting liquid to readily penetrate through its thickness. A suitable bodyside liner 144 may be manufactured from a wide selection of web materials, such as porous foams, reticulated foams, apertured plastic films, natural fibers (for example, wood or cotton fibers), synthetic fibers (for example, polyester or polypropylene fibers), or a combination of natural and synthetic fibers. The bodyside liner 144 is suitably employed to help isolate the wearer's skin from liquids held in the absorbent core 128.

Various woven and nonwoven fabrics can be used for the bodyside liner 144. For example, the bodyside liner may be composed of a meltblown or spunbonded web of polyolefin fibers. The bodyside liner 144 may also be a bonded-carded web composed of natural and/or synthetic fibers. The bodyside liner 144 may be composed of a substantially hydrophobic material, and the hydrophobic material may optionally be treated with a surfactant or otherwise processed to impart a desired level of wettability and hydrophilicity. In a particular embodiment of the present invention, the bodyside liner 144 comprises a nonwoven, spunbond, polypropylene fabric composed of about 2.8-3.2 denier fibers formed into a web having a basis weight of about 20 grams per square meter and a density of about 0.13 grams per cubic centimeter. The fabric may be surface treated with about 0.3 weight percent of a surfactant commercially available from Hodgson Textile Chemicals, Inc. under the trade designation AHCOVEL Base N-62. The surfactant may be applied by any conventional means, such as spraying, printing, brush coating or the like. The surfactant may be applied to the entire bodyside liner 144 or may be selectively applied to particular sections of the bodyside liner 144, such as the medial section along the longitudinal centerline of the diaper, to provide greater wettability of such sections. The bodyside liner 144 may further include a composition applied thereto that is configured to be transferred to the wearer's skin for improving the skin health of the wearer. Suitable compositions for use on the bodyside liner 144 are described in U.S. Patent No. 6,149,934 issued November 21, 2000 to Krzysik et al.

The absorbent core 128 of the diaper 120, as representatively illustrated in FIG. 24, may suitably include a matrix of hydrophilic fibers, such as a web of cellulosic fluff, mixed with particles of a high-absorbency material commonly known as superabsorbent material. In a particular aspect, the absorbent core 128 includes a matrix of cellulosic fluff such as wood pulp fluff and superabsorbent hydrogel-forming particles. The wood pulp fluff may be exchanged with synthetic, polymeric, meltblown fibers or with a combination of meltblown fibers and natural fibers. The superabsorbent particles may be substantially homogeneously mixed with the hydrophilic fibers or may be nonuniformly mixed. The fluff and superabsorbent particles may also be selectively placed into desired zones of the absorbent core 128 to better contain and absorb body exudates. The concentration of the superabsorbent particles may also vary through the thickness of the absorbent core 128. Alternatively, the absorbent core 128 may include a laminate of fibrous webs and superabsorbent material or other suitable means of maintaining a superabsorbent material in a localized area.

The absorbent core 128 may have any of a number of shapes. For example, the absorbent core may be rectangular, I-shaped, or T-shaped. It is generally preferred that the absorbent core 128 be narrow in the crotch region 126 of the diaper 120. It has been found that the absorbent core 128 of the present invention is particularly useful when the width dimension in the crotch region 126 is from about 2.5 to about 12.7 centimeters (1.0 to about 5.0 inches), desirably no more than about 7.6 centimeters (3.0 inches) and more desirably no more than about 5.1 centimeters (2.0 inches). The narrow crotch width dimension of the absorbent core 128 allows the absorbent core 128 to better fit between the legs of the wearer. The size and the absorbent capacity of the absorbent core 128 should be compatible with the size of the intended wearer and the liquid loading imparted by the intended use of the absorbent article.

The high-absorbency material can be selected from natural, synthetic, and modified natural polymers and materials. The high-absorbency materials can be inorganic materials, such as silica gels, or organic compounds, such as crosslinked polymers. The term "crosslinked" refers to any means for effectively rendering normally water-soluble materials substantially water insoluble but swellable. Such means can include, for example, physical entanglement, crystalline domains, covalent bonds, ionic complexes and associations, hydrophilic associations such as hydrogen bonding, and hydrophobic associations or Van der Waals forces.

Examples of synthetic, polymeric, high-absorbency materials include the alkali metal and ammonium salts of poly(acrylic acid) and poly(methacrylic acid), poly(acrylamides), poly(vinyl ethers), maleic anhydride copolymers with vinyl ethers and alpha-olefins, poly(vinyl pyrolidone), poly(vinyl morpholinone), poly(vinyl alcohol), and mixtures and copolymers thereof. Further polymers suitable for use in the absorbent core 128 include natural and modified natural polymers, such as hydrolyzed acrylonitrile-grafted starch, acrylic acid grafted starch, methyl cellulose, carboxymethyl cellulose, hydroxypropyl cellulose, and the natural gums, such as alginates, xanthan gum, locust bean gum, and the like. Mixtures of natural and wholly or partially synthetic absorbent polymers can also be useful in the present invention. Such high-absorbency materials are well known to those skilled in the art and are widely commercially available. Examples of superabsorbent polymers suitable for use in the present invention are SANWET IM 3900 polymer available from Hoechst Celanese located in Portsmouth, Virginia, DOW DRYTECH 2035LD polymer available from Dow Chemical Co. located in Midland, Michigan and Stockhausen W65431 polymer available from Stockhausen Inc., located in Greensboro, NC.

The high absorbency material may be in any of a wide variety of geometric forms. As a general rule, it is preferred that the high absorbency material be in the form of discrete particles. However, the high absorbency material may also be in the form of fibers, flakes, rods, spheres, needles, or the like. As a general rule, the high absorbency material is present in the absorbent core 128 in an amount of from about 5 to about 90 weight percent based on total weight of the absorbent core 128.

Optionally, a substantially hydrophilic tissue wrapsheet (not illustrated) may be employed to help maintain the integrity of the airlaid fibrous structure of the absorbent core 128. The tissue wrapsheet is typically placed about the absorbent core 128 over at least the two major facing surfaces thereof and composed of an absorbent cellulosic material, such as creped wadding or a high wet-strength tissue. In one aspect of the invention, the tissue wrapsheet can be configured to provide a wicking layer which helps to rapidly distribute liquid over the mass of absorbent fibers comprising the absorbent core 128. The wrapsheet material on one side of the absorbent fibrous mass may be bonded to the wrapsheet located on the opposite side of the fibrous mass to effectively entrap the absorbent core 128.

As representatively illustrated in FIG. 24, the disposable diaper 120 may include a pair of containment flaps 156 that are configured to provide a barrier to the lateral flow of body exudates. The containment flaps 156 may be located along the laterally opposed side edges 130 of the diaper adjacent the side edges of the absorbent core 128. Each containment flap 156 typically defines an unattached edge which is configured to maintain an upright, perpendicular configuration in at least the crotch region 126 of the diaper 120 to form a seal against the wearer's body. The containment flaps 156 may extend longitudinally along the entire length of the absorbent core 128 or may only extend partially along the length of the absorbent core 128. When the containment flaps 156 are shorter in length than the absorbent core 128, the containment flaps 156 can be selectively positioned anywhere along the side edges 130 of diaper 120 in the crotch region 126. In a particular aspect of the invention, the containment flaps 156 extend along the entire length of the absorbent core 128 to better contain the body exudates.

Such containment flaps 156 are generally well known to those skilled in the art. For example, suitable constructions and arrangements for containment flaps 156 are described in U.S. Patent 4,704,116 issued November 3, 1987, to K. Enloe.

The diaper 120 may further include elastics at the waist edges 132 and side edges 130 of the diaper 120 to further prevent leakage of body exudates and support the absorbent core 128. For example, as representatively illustrated in FIG. 24, the diaper 120 of the present invention may include a pair of leg elastic members 154 which are connected to the laterally opposed side edges 130 of the diaper 120 in the crotch region 126. The diaper 120 may also include a pair of waist elastic members 158 which are connected to the longitudinally opposed waist edges 132 of the diaper 120. The leg elastics 154 and waist elastics 158 are generally adapted to fit about the legs and waist of a wearer in use to maintain a positive, contacting relationship with the wearer to effectively reduce or eliminate the leakage of body exudates from the diaper 120.

Materials suitable for use as the leg elastics 154 and waist elastics 158 are well known to those skilled in the art. Exemplary of such materials are sheets or strands or ribbons of a polymeric, elastomeric material which are adhered to the outer cover 142 in a stretched position, or which are attached to the outer cover 142 while the outer cover is pleated, such that elastic constrictive forces are imparted to the outer cover 142. The leg elastics 154 may also include such materials as polyurethane, synthetic and natural rubber.

The different aspects of the present invention advantageously provide flexible, cloth-like fasteners 160. The mechanical fastener 160 is provided on a thin flexible layer 162 with the mechanical fastening material 166 embedded therein. This configuration provides a mechanical fastener 160 which may be bent or conformed and yet provides reliable securement of the article about the wearer. Moreover, the perimeter edge 170 of the mechanical fastening material 166 is surrounded by the flexible layer 162 while being recessed within the flexible layer 162 thereby reducing the possibility of irritation or red-marking. Further, the mechanical fastener 160 of the present invention is provided with multiple first discrete islands 164 of mechanical fastener material 166 and multiple second discrete islands 165 of foam fastener material. As such, the flexibility of the mechanical fastener 160 is additionally supplemented by providing areas of flexible material between the islands of fastener material 166. This specially located flexible material may be bent instead of the more rigid fastener material. Accordingly, the possibility of creasing the mechanical fastener material 166 is also reduced, thereby further reducing the possibility of irritation caused by any rigid edges of the mechanical fastener material 166 coming into contact with the wearer's skin.

The mechanical fastener 160 of the present invention may be provided in combination with a disposable absorbent article. As a result, the absorbent article advantageously provides a fastener 160 that enhances the comfort of the wearer by reducing the opportunity for red-marking and irritation. In addition, the increased flexibility of the fasteners 160 of the present invention allows the fasteners 160 to better accommodate the movement of particularly the active wearer, thereby providing more reliable securement of the article about a wearer. The fit and comfort of the article are also similarly enhanced as the flexible fastener may be adjusted to a wider range of positions in use, to achieve the optimum fastening location upon the wearer.

While the invention has been described in detail with respect to specific embodiments, it will be appreciated that there are variations of, and equivalents to these embodiments. Accordingly, the scope of the present invention should be determined by the appended claims.

## Claims

1. A mechanical fastener (160) comprising:
a) a flexible layer (162);
b) a plurality of first discrete fastener islands (164) having a mechanical fastening material (166) and a backing material (168) having a first surface attached to the mechanical fastening material (166) and a second surface attached to the flexible layer (162) wherein the mechanical fastening material (166) is a hook material; and **characterised in that** the mechanical fastener further comprises:
c) a plurality of second discrete fastener islands (165) comprising a foam fastening layer that is attached to the flexible layer (162) and includes a surface having a plurality of free-standing struts.

2. The mechanical fastener of claim 1 at least some of the free-standing struts including a surface modifier.

3. The mechanical fastener of claim 2, wherein the surface modifier includes a polyethylene polymer.

4. The mechanical fastener of claim 3, wherein the surface modifier includes a mixture of the polyethylene polymer and a blend of copolymers.

5. The mechanical fastener of any preceding claim wherein the first discrete fastener islands (164) have a generally circular shape.

6. The mechanical fastener of any preceding claim wherein the flexible layer (162) is extensible.

7. The mechanical fastener of any preceding claim wherein the fastener further defines a user's end (182) and a bond end (184) wherein the bond end (184) is permanently attached to a disposable absorbent article (120) and the users end contains the discrete fastener islands (164,165) and is configured to secure the disposable absorbent article about a wearer.

8. The mechanical fastener of any preceding claim wherein the flexible layer is a foam layer.

9. The mechanical fastener of any preceding claim wherein said second fastener islands (165) are located closer to the edge of the fastener than said first fastener islands (164).

10. A disposable absorbent article (120) comprising:
a) an outer cover (142);
b) a bodyside liner (144);
c) an absorbent core (128) located between the bodyside liner (144) and the outer cover (142); and
d) at least one mechanical fastener (160) in accordance with any of claims 1 to 6.

11. The disposable absorbent article of claim 10 wherein the mechanical fastener (160) is configured to refastenably engage directly to the outer cover (142).

12. The disposable absorbent article of claim 11 and further comprising an attachment panel (186) wherein the mechanical fastener (160) is configured to refastenably engage the attachment panel (186).

## Patentansprüche

1. Mechanischer Befestiger (160), welcher umfasst:
a) eine flexible Schicht (162);
b) eine Vielzahl von ersten diskreten Befestigerinseln (164), welche ein mechanisches Befestigungsmaterial (166) und eine Rückschicht (168) aufweisen, welche eine erste Oberfläche aufweist, welche an dem mechanischen Befestigungsmaterial (166) befestigt ist, und eine zweite Oberfläche aufweist, welche an der flexiblen Schicht (162) befestigt ist, wobei das mechanische Befestigungsmaterial (166) ein Hakenmaterial ist; und **dadurch gekennzeichnet, dass** der mechanische Befestiger des Weiteren umfasst:
c) eine Vielzahl von zweiten diskreten Befestigerinseln (165), die eine Schaumstoffbefestigungsschicht umfassen, die an der flexiblen Schicht (162) befestigt ist und eine Oberfläche beinhaltet, die eine Vielzahl von freistehenden Streben aufweist.

2. Mechanischer Befestiger gemäß Anspruch 1, wobei zumindest einige der freistehenden Streben ein Oberflächenmodifizierungsmittel beinhalten.

3. Mechanischer Befestiger gemäß Anspruch 2, wobei das Oberflächenmodifizierungsmittel Polyethylenpolymer beinhaltet.

4. Mechanischer Befestiger gemäß Anspruch 3, wobei das Oberflächenmodifizierungsmittel eine Mischung aus dem Polyethylenpolymer und einer Mischung von Copolymeren beinhaltet.

5. Mechanischer Befestiger gemäß einem der vorherigen Ansprüche, wobei die ersten diskreten Befestigerinseln (164) eine generell kreisförmige Form aufweisen.

6. Mechanischer Befestiger gemäß einem der vorherigen Ansprüche, wobei die flexible Schicht (162) erweiterbar ist.

7. Mechanischer Befestiger gemäß einem der vorherigen Ansprüche, wobei der Befestiger des Weiteren ein Benutzerende (182) und ein Verbindungsende (184) definiert, wobei das Verbindungsende (184) dauerhaft an einem absorptionsfähigen Einweg-Artikel (120) befestigt ist und das Benutzerende die diskreten Befestigerinseln (164, 165) enthält und eingerichtet ist, den absorptionsfähigen Einweg-Artikel um einen Träger zu fixieren.

8. Mechanischer Befestiger gemäß einem der vorherigen Ansprüche, wobei die flexible Schicht eine Schaumstoffschicht ist.

9. Mechanischer Befestiger gemäß einem der vorherigen Ansprüche, wobei die zweiten Befestigerinseln (165) näher an der Kante des Befestigers angeordnet sind als die ersten Befestigerinseln (164).

10. Absorptionsfähiger Einweg-Artikel (120), welcher umfasst:
a) eine äußere Deckschicht (142);
b) eine körperseitige Auskleidung (144);
c) einen absorptionsfähigen Kern (128), der zwischen der körperseitigen Auskleidung (144) und der äußeren Deckschicht (142) angeordnet ist; und
d) mindestens einen mechanischen Befestiger (160) gemäß einem der Ansprüche 1 bis 6.

11. Der absorptionsfähige Einweg-Artikel gemäß Anspruch 10, wobei der mechanische Befestiger (160) eingerichtet ist, sich wiederverschließbar direkt mit der äußeren Deckschicht (142) zu verbinden.

12. Der absorptionsfähige Einweg-Artikel gemäß Anspruch 11 und welcher des Weiteren eine Befestigungsbahn (186) umfasst, wobei der mechanische Befestiger (160) eingerichtet ist, sich wiederverschließbar mit der Befestigungsbahn (186) zu verbinden.

## Revendications

1. Attache mécanique (160) comprenant :
a) une couche flexible (162) ;
b) une pluralité de premiers îlots d'attache individualisés (164) ayant un matériau d'attache mécanique (166) et un matériau formant dossier (168) ayant une première surface fixée au matériau d'attache mécanique (166) et une seconde surface fixée à la couche flexible (162), le matériau d'attache mécanique (166) étant un matériau à crochets ;
l'attache mécanique étant **caractérisée en ce qu'**elle comprend, en outre :
c) une pluralité de seconds îlots d'attache individualisés (165) comprenant une couche d'attache en mousse qui est fixée à la couche flexible (162) et inclut une surface ayant une pluralité de soutiens autonomes.

2. Attache mécanique selon la revendication 1, dans laquelle au moins certains soutiens autonomes incluent un modificateur de surface.

3. Attache mécanique selon la revendication 2, dans laquelle le modificateur de surface inclut un polymère polyéthylène.

4. Attache mécanique selon la revendication 3, dans laquelle le modificateur de surface inclut un mélange de polymère polyéthylène et de mélange de copolymères.

5. Attache mécanique selon l'une quelconque des revendications précédentes, dans laquelle les premiers îlots d'attache individualisés (164) ont une forme généralement circulaire.

6. Attache mécanique selon l'une quelconque des revendications précédentes, dans laquelle la couche flexible (162) est extensible.

7. Attache mécanique selon l'une quelconque des revendications précédentes, ladite attache définissant, en outre, une extrémité utilisateur (182) et une extrémité de liaison (184), dans laquelle l'extrémité de liaison (184) est fixée à demeure à un article absorbant jetable (120) et l'extrémité utilisateur (182) contient les îlots d'attache individualisés (164,165) et est configurée pour immobiliser l'article absorbant jetable autour d'un porteur.

8. Attache mécanique selon l'une quelconque des revendications précédentes, dans laquelle la couche flexible est une couche de mousse.

9. Attache mécanique selon l'une quelconque des revendications précédentes, dans laquelle lesdits seconds îlots d'attache (165) sont situés plus près du bord de l'attache que lesdits premiers îlots d'attache (164).

10. Article absorbant jetable (120) comprenant :
a) une feuille extérieure de couverture (142) ;
b) une doublure côté corporel (144) ;
c) un noyau absorbant (128) situé entre la doublure côté corporel (144) et la feuille extérieure de couverture (142) ; et
d) au moins une attache mécanique (160) selon l'une quelconque des revendications 1 à 6.

11. Article absorbant jetable selon la revendication 10, dans lequel l'attache mécanique (160) est configurée pour venir en prise repositionnable directement avec la feuille extérieure de couverture (142).

12. Article absorbant jetable selon la revendication 11 et comprenant, en outre, un panneau de fixation (186), article dans lequel l'attache mécanique (160) est configurée pour venir en prise repositionnable avec le panneau de fixation (186).
